# EUROPEAN PATENT SPECIFICATION

(11) **EP 4 351 577 B1**
(45) Date of publication and mention of the grant of the patent: **11.11.2026**
(21) Application number: 22732509.9
(22) Date of filing: 07.06.2022
(51) Int. Cl.: A61K 31/495, A61K 31/4184, A61K 31/4523, A61K 45/06, A61P 35/00, A61K 31/502, A61K 31/44, A61K 31/517

(54) **COMBINATION THERAPY FOR CANCER TREATMENT**
KOMBINATIONSTHERAPIE ZUR KREBSBEHANDLUNG
POLYTHÉRAPIE POUR LE TRAITEMENT DU CANCER

(30) Priority: 09.06.2021 EP 21178403
(43) Date of publication of application: 17.04.2024
(73) Proprietor: F. Hoffmann-La Roche AG, 4070 Basel (CH); CHUGAI SEIYAKU KABUSHIKI KAISHA, Tokyo 115-8543 (JP)
(72) Inventor: ECKMANN, Jan, 82377 Penzberg (DE); FRIESS, Thomas, 82377 Penzberg (DE); HERTING, Frank, 82377 Penzberg (DE); IDE, Yusuke, Kamakura-shi, Kanagawa 247-8530 (JP); TANAKA, Hiroshi, Kamakura-shi, Kanagawa 247-8530 (JP); PETTAZZONI, Piergiorgio Francesco Tommaso, 4070 Basel (CH); WICHMANN, Juergen, 4070 Basel (CH)
(74) Representative: Vitra, Hermeto
(86) International application number: PCT/EP2022/065393
(87) International publication number: WO 2022/258612

(56) References cited:
- WO-A1-2020/261156
- WO-A1-2021/116055
- WO-A1-2022/019329
- ZAREMBA A ET AL: "Malignes Melanom", ONKOLOGE, SPRINGER, BERLIN, DE, vol. 26, no. 8, 27 April 2020 (2020-04-27), pages 713 - 720, XP037200383, ISSN: 0947-8965, [retrieved on 20200427], DOI: 10.1007/S00761-020-00762-1
- WICHMANN JÜRGEN ET AL: "Preclinical Characterization of a Next-Generation Brain Permeable, Paradox Breaker BRAF Inhibitor", CLINICAL CANCER RESEARCH, vol. 28, no. 4, 15 February 2022 (2022-02-15), US, pages 770 - 780, XP055950206, ISSN: 1078-0432, Retrieved from the Internet <URL:https://watermark.silverchair.com/770.pdf?token=AQECAHi208BE49Ooan9kkhW_Ercy7Dm3ZL_9Cf3qfKAc485ysgAAAuEwggLdBgkqhkiG9w0BBwagggLOMIICygIBADCCAsMGCSqGSIb3DQEHATAeBglghkgBZQMEAS4wEQQMHJBfkqeXkG0HY-9KAgEQgIIClFz3qtMpSCAdQkBcgRveHv0OQufHjuC7OEDWOF9ItRb3DEe37LWGyzCnrWbwWKTE50Hmk_AW0znmhAfizf7-wrTe8iHd6u8K> DOI: 10.1158/1078-0432.CCR-21-2761

## Description

The present invention relates to a combination of a BRAF inhibitor and a MEK inhibitor, as well as uses and pharmaceutical compositions thereof, wherein the BRAF inhibitor is a compound of formula (I) 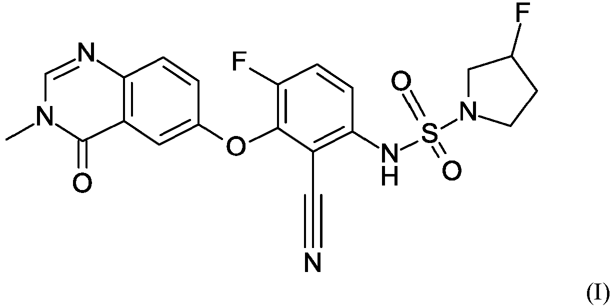
or a pharmaceutically acceptable salt or solvate thereof, and
wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or pharmaceutically acceptable salts thereof.

The present invention provides in particular a BRAF inhibitor and a MEK inhibitor for use in the treatment of cancer, wherein the BRAF inhibitor is a compound of formula (I) 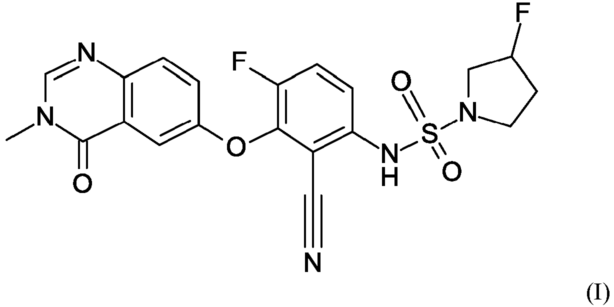
or a pharmaceutically acceptable salt or solvate thereof, and
wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or pharmaceutically acceptable salts thereof.

Mutant BRAF is a targetable oncogenic driver and three BRAF inhibitors (BRAFi) up to date (Vemurafenib, Dabrafenib Encorafenib) reached the market showing efficacy in BRAFV600E-positive melanoma. However, rapid acquisition of drug resistance is almost universally observed and the duration of the therapeutic benefits for the targeted therapy remains limited.

Moreover, the developed first generation BRAF inhibitors revealed an unexpected and "paradoxical" ability to repress MAPK signalling in BRAF^{V600E}-driven tumors while the same inhibitors presented MAPK stimulatory activities in BRAF wild type (WT) models (N Engl J Med 2012; 366:271-273; and British Journal of Cancer volume 111, pages640-645(2014)).

Mechanistic studies on the RAF paradox then clarified that oncogenic BRAF^{V600E} phosphorylates MEK 1/2 in its monomeric cytosolic form while WT BRAF and RAF1 activation requires a complex step of events including cell membrane translocation and homo and/or heterodimerization promoted by activated RAS (KRAS, NRAS, HRAS) (Nature Reviews Cancer volume 14, pages455-467(2014)).

The binding of first generation BRAF inhibitors like Vemurafenib, Dabrafenib and Encorafenib to a WT BRAF or RAF1 protomer, quickly induces RAF homo and/or hetero dimerization and membrane association of the newly formed RAF dimer. In the dimeric conformation, one RAF protomer allosterically induces conformational changes of the second resulting in a kinase active status and, importantly, in a conformation unfavourable for the binding of the inhibitor. The dimer induced by drug treatment, as a result, promotes MEK phosphorylation by the catalysis operated by the unbound protomer with hyperactivation of the pathway.

Tumors almost inevitable evade from BRAFi treatment and the mechanism in the vast majority of cases involve the acquired ability of triggering RAF dimerization. This effect can be counteracted by MEK inhibitor (MEKi) combined treatment. These agents, however, present very poor therapeutic index which limits MEKi achievable doses in human. As a result, resistance to the combination therapy of a first generation BRAFi with a MEKi is still mediated by RAF paradoxical activation. The clinical benefit of those combination therapies remains thus limited.

WO2020/261156 discloses BRAF inhibitors with a quinazolinone scaffold in combination with a MEK inhibitor and for the treatment of cancer.

The present invention relates to a new combination of a BRAF inhibitor of formula (I) and a MEK inhibitor for use in the treatment of cancer, in particular of melanoma. The compound of formula (I) is a BRAF inhibitor, which is showing neglectible paradoxical activation of the MAPK signaling pathway (paradox breaker) when compared to the first generation BRAF inhibitors that are on the market: Encorafenib, Dabrafenib and Vemurafenib (paradox inducers). In addition to this property, the compound of formula (I) also has very potent brain penetration properties, thus providing an urgently needed alternative therapy for the treatment of cancers which metastasized in the brain. The present invention discloses a new combination for use in cancer therapy with strong combined activity on BRAF associated tumours with the potential to overcome the rapidly acquired treatment resistance frequently observed in patients treated with first generation BRAF inhibitors. The combination as disclosed in the present invention for use in the treatment of cancer, presents unexpected combination activity that go far beyond the additive effects of MEKi and BRAFi monotherapies.

### Brief description of Figures:

**Figure 1** discloses the P-ERK inhibition by (3*R*)-*N*-[2-cyano-4-fluoro-3-(3-methyl-4-oxo-quinazolin-6-yl)oxy-phenyl]-3-fluoro-pyrrolidine-1-sulfonamide (herein referred to as Compound Ia) in combination with the MEK inhibitor Cobimetinib in the cell line A375 BRAF/NRAS when compared with the first generation BRAF inhibitor Encorafenib.
**Figure 2** discloses the growth inhibition by Compound Ia in combination with the MEK inhibitor Cobimetinib in the cell line A375 BRAF/NRAS when compared with the first generation BRAF inhibitor Encorafenib.
**Figure 3** discloses that the combination of Compound Ia with Cobimetinib results in a drastic and synergistic reduction of on the tumour volume of mice which were implanted the cell line A375 NRAS when compared with the monotherapies.
**Figure 4** discloses a clear synergistic effect of Compound Ia in combination with Binimetinib on the tumour volume of mice which were implanted the cell line A375 NRAS when compared with the first generation BRAF inhibitor Encorafenib in combination with Binimetinib.
**Figure 5** discloses a clear dose-dependent synergistic effect of Compound Ia in combination with 2-(4-cyclopropyl-2-fluoroanilino)-3,4-difluoro-5-[[3-fluoro-2-(methylsulfamoylamino)pyridin-4-yl]methyl]benzamide sodium salt (herein referred to as Compound IIa) on the tumour volume of mice which were implanted the cell line A375 NRAS when compared with the monotherapies.
**Figure 6** discloses a clear synergistic effect of Encorafenib in combination with Compound IIa on the growth inhibition in the cell line A375. Figure 6 is not according to the invention and is present for illustration purposes only.

The term "inhibitor" denotes a compound which competes with, reduces or prevents the binding of a particular ligand to particular receptor, or which reduces or prevents the function of a particular protein. In particular, an inhibitor as used therein refers to compounds which target, decrease or inhibit activity of the respective target selected from BRAF and MEK, particular inhibitors have an IC50 value below 1 µM, below 500 nM, below 200 nM, below 100 nM, below 50 nM, below 25 nM, below 10 nM, below 5 nM, 2 nM or below 1 nM. In some embodiments of the invention the term "BRAF inhibitor" refers to compounds that decrease BRAF kinase activity at least about 10%, at least about 20%, at least about 30%, at least about 40%, at least about 50%, at least about 60%, at least about 70%, at least about 75%, at least about 80%, at least about 85%, at least about 90%, at least about 95% or at least about 99%.. In some embodiments of the invention the term "MEK inhibitor" refers to compounds that decrease MEK kinase activity at least about 10%, at least about 20%, at least about 30%, at least about 40%, at least about 50%, at least about 60%, at least about 70%, at least about 75%, at least about 80%, at least about 85%, at least about 90%, at least about 95% or at least about 99%.

The term "IC50" refers to the concentration of a particular compound required to inhibit 50% of a specific measured activity.

The term "pharmaceutically acceptable salt" refers to those salts of the compound of formula (I) or of the MEK inhibitor which retain the biological effectiveness and properties of the free bases or free acids, which are not biologically or otherwise undesirable. These salts can for instance be formed with inorganic acids such as hydrochloric acid, hydrobromic acid, sulfuric acid, nitric acid, phosphoric acid, in particular hydrochloric acid, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, N-acetylcystein. In addition, these salts may be prepared by addition of an inorganic base or an organic base to the free acid. Salts derived from an inorganic base include, but are not limited to, the sodium, potassium, lithium, ammonium, calcium, magnesium salts. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, lysine, arginine, N-ethylpiperidine, piperidine, polyimine resins. Particular pharmaceutically acceptable salts of a compound of formula (I) are the hydrochloride salts, methanesulfonic acid salts and citric acid salts. Particular pharmaceutically acceptable salts of [3,4-difluoro-2-(2-fluoro-4-iodoanilino)phenyl]-[3-hydroxy-3-[(2S)-piperidin-2-yl]azetidin-1-yl]methanone are the fumarate salts and succinate salts, in particular hemifumarate salts and hemisuccinate salts. Particular pharmaceutically acceptable salts of a compound of formula (II) are the alkali metal salts such as lithium salts, sodium salts, potassium salts, cesium salts and rubidium salts, and sodium salts and potassium salts are preferred.

The term "solvate" refers to non-covalent stoichiometric or nonstoichiometric combinations of solvent and solute. The term "hydrate" refers to non-covalent stoichiometric or nonstoichiometric combinations of water and solute. For example, compounds of formula (I) and pharmaceutically acceptable salts thereof, can exist in unsolvated as well as solvated forms with pharmaceutically acceptable solvents such as anisole, dichloromethane, toluene, 1,4-dioxane, water.

The compound of formula (I) contains one asymmetric center and can be present in the form of optically pure enantiomers or mixtures of enantiomers such as, for example, racemates.

According to the Cahn-Ingold-Prelog Convention the asymmetric carbon atom can be of the "R" or "S" configuration.

In one aspect, the present invention provides a BRAF inhibitor and a MEK inhibitor for use in the treatment of cancer, wherein the BRAF inhibitor is a compound of formula (I) 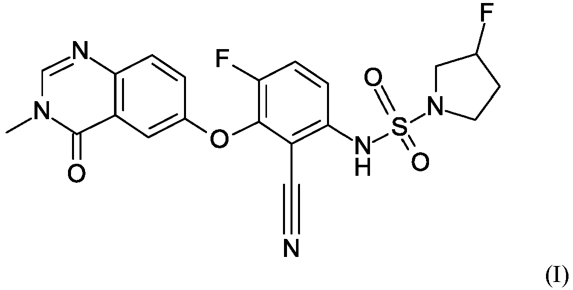 or a pharmaceutically acceptable salt or solvate thereof, wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or pharmaceutically acceptable salts thereof.

In some embodiments of the present invention the compound of formula (I) is a compound according to formula (Ia)

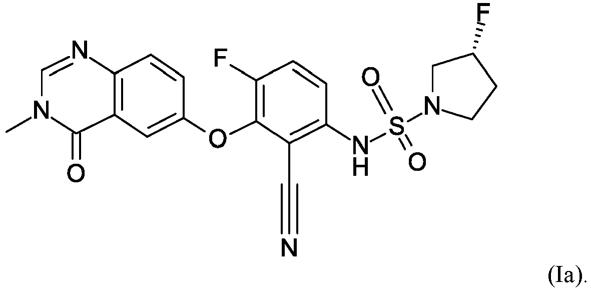

In some embodiments of the present invention the compound of formula (I) is a compound according to formula (Ib)

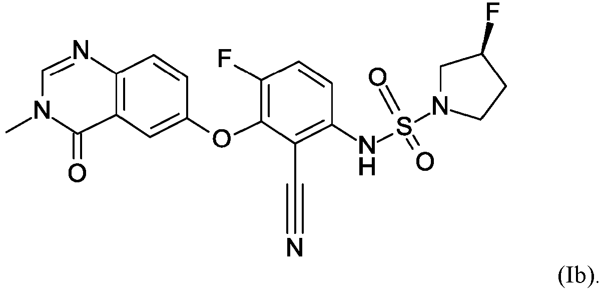

Non-limiting examples of MEK inhibitors include 2-(4-cyclopropyl-2-fluoroanilino)-3,4-difluoro-5-[[3-fluoro-2-(methylsulfamoylamino)pyridin-4-yl]methyl]benzamide, cobimetinib, binimetinib, trametinib, selumetinib, pimasertib, refametinib, N-[2(R),3-dihydroxypropoxy]-3,4-difluoro-2-(2-fluoro-4-iodophenylamino)benzamide (PD-325901), 2-(2-chloro-4-iodophenylamino)-N-(cyclopropylmethoxy)-3,4-difluorobenzamide (Cl-1040) and 3-[2(R),3-dihydroxypropyl]-6-fluoro-5-(2-fluoro-4-iodophenylamino)-8-methylpyrido[2,3-d]pyrimidine-4,7(3H,8H)-dione (TAK-733).

In some embodiments of the present invention the MEK inhibitor is a compound of formula (II) 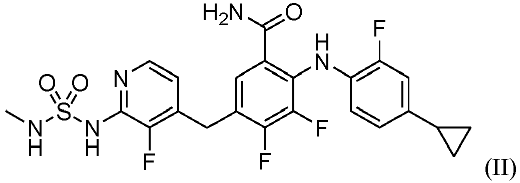 or a pharmaceutically acceptable salt or solvate thereof.

The compound of formula (II) is an orally available MEK inhibitor having potent MEK-inhibiting activity and high RAF/MEK complex-stabilizing activity. The chemical name of formula (II) is 2-(4-cyclopropyl-2-fluoroanilino)-3,4-difluoro-5-[[3-fluoro-2-(methylsulfamoylamino)pyridin-4-yl]methyl]benzamide.

In some embodiments of the present invention the compound of formula (II) is a sodium salt according to formula (IIa)

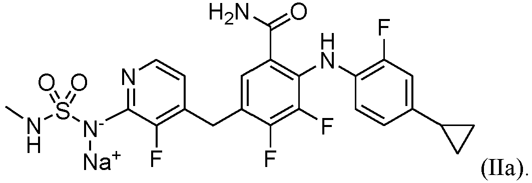

In some embodiments of the present invention the MEK inhibitor is cobimetinib. Cobimetinib is an orally available, potent and highly selective inhibitor of MEK1 and MEK2, central components of the RAS/RAF pathway. Cobimethib has the chemical name [3,4-difluoro-2-(2-fluoro-4-iodoanilino)phenyl]-[3-hydroxy-3-[(2S)-piperidin-2-yl]azetidin-1-yl]methanone and has the following structure:

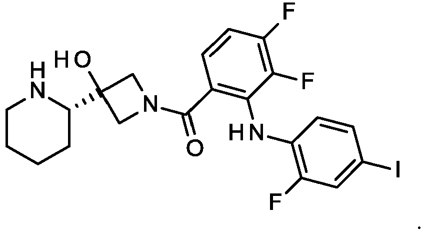

Cobimetinib may be prepared following the methods described in WO 2007/044515. Cobimetinib is commercially available and has the following CAS Registry Number: 934660-93-2.

In some embodiments of the present invention the MEK inhibitor is binimetinib. Binimetinib is an orally available, potent and highly selective inhibitor of MEK1 and MEK2, central components of the RAS/RAF pathway. Binimetinib has the chemical name 5-[(4-bromo-2-fluorophenyl)amino]-4-fluoro-N-(2-hydroxyethoxy)-1-methyl-1H-benzimidazole-6-carboxamide and has the following structure:

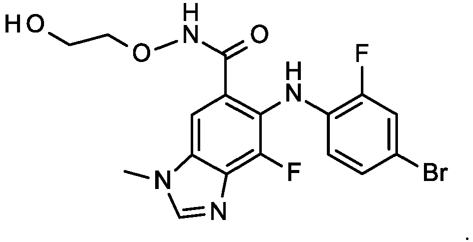

Binimetinib may be prepared following the methods described in WO 2003/077914. Binimetinib is commercially available and has the following CAS Registry Number: 606143-89-9.

### Assay procedures

### Materials

DMEM no-phenol red medium supplemented with L-glutamine was purchased from (Thermo Fisher Scientific). Fetal bovine serum (FBS) was purchased from VWR. Advanced ERK phospho-T202 /Y204 kit - 10,000 tests was purchased from Cisbio cat# 64AERPEH. A375 were originally obtained from ATCC and banked by the Roche repository. 384-well microplates were purchased from Greiner Bio-One, 384-well, (With Lid, HiBase, Low volume cat 784-080).

### HTRF assay for P-ERK determination in A375 cells

A375 is a cellular cancer model expressing V600E mutated BRAF. ERK 1,2 phosphorylation (terminal member of the phosphorylation cascade of the MAPK pathway) is hereafter reported as main readout for the activation status of the MAPK pathway. Prior to the assay, the A375 cell line is maintained in DMEM no-phenol red medium supplemented with 10% fetal bovine serum (FBS). Following compound treatment, P-ERK levels are determined by measuring FRET fluorescence signal induced by selective binding of 2 antibodies provided in the mentioned kit (Cisbio cat# 64AERPEH) on ERK protein when phosphorylated at Thr202/Tyr204. Briefly, 8000 cells/well in 12 µl media/well are plated in the 384-well plate and left overnight in the incubator (at 37 °C with 5% CO2-humidified atmosphere), the following day the plate is treated in duplicate with test compounds, dabrafenib and PLX8394 (the latter two as controls) at the following final drug concentrations: 10µM-3µM-1µM-0.3µM-0.1µM-0.03µM-0,01µM-0.003µM-0.001µM, all wells are subjected to DMSO normalization and drug incubation occurs for 1 hour. Then, 4µl of a 4X lysis buffer supplied with the kit are added to the wells, the plate is then centrifuged for 30 second (300 rcf) and incubated on a plate shaker for 1h at RT.

At the end of the incubation 4µL/well of advanced P-ERK antibody solution (prepared according to manufacturer's instruction) followed by 4µL/well of criptate P-ERK antibody solution (prepared according to manufacturer's instruction) (Cisbio cat# 64AERPEH) are added to test wells.

In order to allow proper data normalization control wells without drug treatment reported are always included in each plate (according to manufacturer's instruction):

### p-ERK HTRF well content of controls and experimental (µl):

| neg ctrl | pos ctrl | neut ctrl | cpd | blank | |
|---|---|---|---|---|---|
| - | - | 12 | 12 | 12 | Cells |
| 12 | - | - | - | - | Media |
| - | - | - | <0.05 | - | Cpd |
| - | 16 | - | - | - | control lysate (ready-to-use) |
| 4 | - | 4 | 4 | 4 | 4x lysis buffer |
| 4 | 4 | 4 | 4 | - | Advanced p-ERK antibody solution |
| - | - | - | - | 4 | Advanced p-ERK1/2 Cryptate antibody solut. |
| 20 | 20 | 20 | 20 | 20 | Total volume in Well |

The plate is then centrifuged at 300 rcf for 30 second, sealed to prevent evaporation and incubated overnight in the dark at room temperature.

The plate is then analyzed and fluorescence emission value collected through a Pherastast FSX (BMG Labtech) apparatus at 665 and 620 nM.

The obtained fluorescence values are processed according to the formula Ratio=Signal(620nm)/Signal(625nm)*10000 then the average of the ratio on the blank is subtracted to all values.

Data are normalized considering the average of the ratio (blank subtracted) derived by DMSO only treated cells as 100% and by considering the average of the ratio (blank subtracted) derived by 10µM dabrafenib treated cells as 0%. Mean of the normalized points are fitted with sigmoidal curve and IC50 determined. The results are summarized in Table 1. Biochemical assays confirmed the high affinity of compounds of formula (Ia) and (Ib) against BRAF and BRAF^{V600E}. Kd is the dissociation constant in the biochemical experiments.

**Table 1: Compounds (Ia) and (Ib) have a high affinity for BRAF and BRAF^{V600E} and high selectivity over C-terminal Src kinase (CSK) and lymphocyte-specific tyrosine protein kinase (LCK). Kd is the dissociation constant in the biochemical experiments. IC50 was measured in A375 cell line as described above.**

| **Compound** | **Kd (µM)** | | | | | **pERK IC50 (µM)** |
|---|---|---|---|---|---|---|
| | **BRAF** | **BRAF V600E** | **CRAF** | **CSK** | **LCK** | **A375** |
| **(Ia)** | 0.0006 | 0.0012 | 0.0017 | 23.3 | 40 | 0.0069 |
| **(Ib)** | 0.0013 | 0.0009 | 0.0012 | 9.16 | 20.12 | 0.0106 |

The preparation of the compound of formula (I) of the present invention may be carried out in sequential or convergent synthetic routes. Syntheses of the invention are shown in the following general scheme. The skills required for carrying out the reactions and purifications of the resulting products are known to those skilled in the art.

In more detail, the compound of formula (I) can be manufactured by the methods given below, by the methods given in the examples or by analogous methods. Appropriate reaction conditions for the individual reaction steps are known to a person skilled in the art. The reaction sequence is not limited to the one displayed in scheme 1, however, depending on the starting materials and their respective reactivity the sequence of reaction steps can be freely altered. Starting materials are either commercially available or can be prepared by methods analogous to the methods given below, by methods described in references cited in the description or in the experimental procedures below, or by methods known in the art.

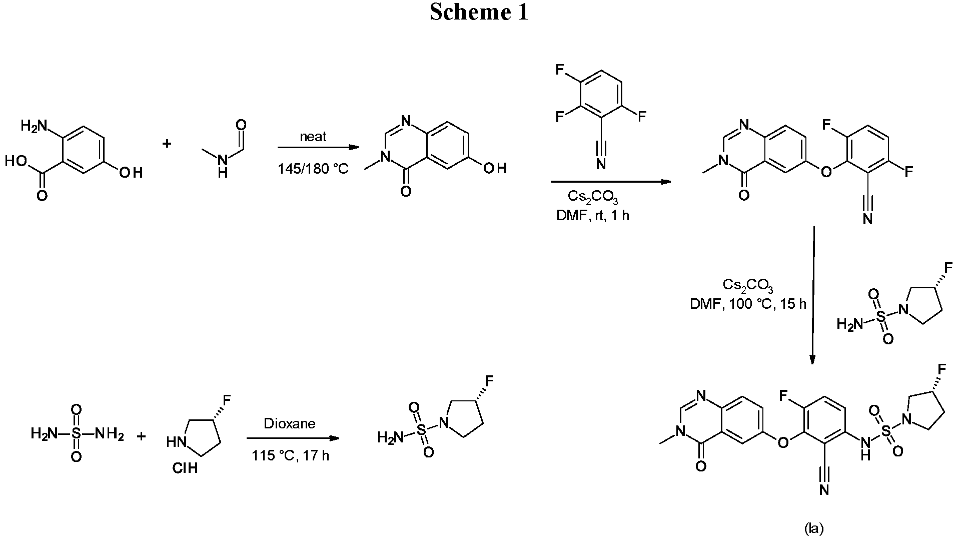

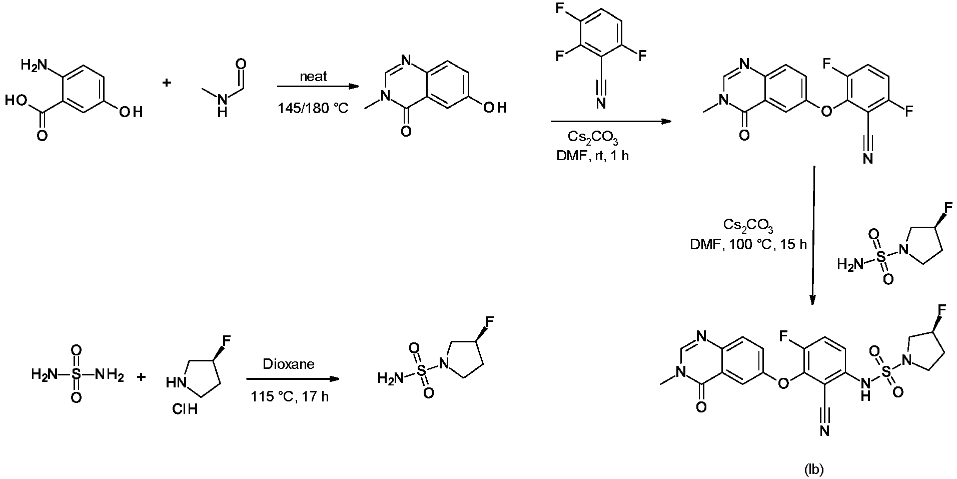

It will be appreciated that the compound of formula (I) in this invention may be derivatised at functional groups to provide derivatives which are capable of conversion back to the parent compound *in vivo.*

### Experimental Procedures

### (3R)-N-[2-cyano-4-fluoro-3-(3-methyl-4-oxo-quinazolin-6-yl)oxy-phenyl]-3-fluoro-pyrrolidine-1-sulfonamide (compound of formula (Ia)) and (3S)-N-[2-cyano-4-fluoro-3-(3-methyl-4-oxo-quinazolin-6-yl)oxy-phenyl]-3-fluoro-pyrrolidine-1-sulfonamide (compound of formula (Ib))

### 6-hydroxy-3-methyl-quinazolin-4-one

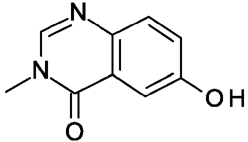

2-Amino-5-hydroxybenzoic acid (10 g, 65.3 mmol, Eq: 1.0) and N-methylformamide (30 g, 29.9 mL, 503 mmol, Eq: 7.7) were heated at 145 °C for 21 h 45 min, then cooled to rt. The reaction mixture was diluted with 50 mL H₂O and stirred at rt for 20 min. The resulting precipitate was collected by filtration. The light brown solid was washed 3 × with 20 mL water. The solid was taken up in toluene and evaporated to dryness (3 ×). The solid was dried in vacuo at 40 °C overnight under high vacuum to give the title compound as a light brown solid (10.3 g, 89% yield). MS (ESI) *m*/*z:* 177.1 [M+H]⁺.

### 3,6-difluoro-2-(3-methyl-4-oxo-quinazolin-6-yl)oxy-benzonitrile

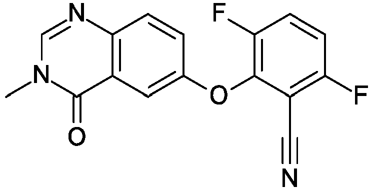

Cesium carbonate (3.22 g, 9.79 mmol, Eq: 1.15) was added at rt to a solution of 6-hydroxy-3-methylquinazolin-4-one (1500 mg, 8.51 mmol, Eq: 1.0) in N,N-dimethylformamide (35 mL). The mixture was stirred for 30 min at rt then 2,3,6-trifluorobenzonitrile (1.47 g, 1.08 ml, 9.37 mmol, Eq: 1.1) was added. After 1 h, the reaction was cooled on ice and diluted with water (120 mL). The resultant solid was collected by filtration, washed with iced water (100 mL) and heptane (100 mL) and suction-dried. The solid was taken up in toluene and evaporated to dryness (3 ×) then dried overnight in vacuo to give the title compound as a light brown solid (2.58 g, 97% yield). MS (ESI) *m*/*z:* 314.1 [M+H]⁺.

### (3R)-3-fluoropyrrolidine-1-sulfonamide

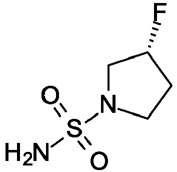

(R)-3-Fluoropyrrolidine hydrochloride (1.8 g, 14.3 mmol, Eq: 1.2) was added to a solution of sulfuric diamide (1.148 g, 11.9 mmol, Eq: 1.0) and triethylamine (2.42 g, 3.33 mL, 23.9 mmol, Eq: 2) in dioxane (10 mL). The reaction was stirred in a sealed tube at 115 °C for 15.5 h then cooled to rt and concentrated in vacuo. The residue was diluted with DCM, evaporated with silica gel to dryness and transferred to a column. Purification by flash chromatography (40 g silica, 80% EtOAc) gave the title compound as a white crystalline solid (1.82 g, 91% yield). MS (ESI) m/z: 169.1 [M+H]⁺.

### (3S)-3-fluoropyrrolidine-1-sulfonamide

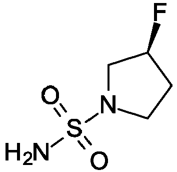

Triethylamine (304 mg, 419 µl, 3.01 mmol, Eq: 2.0) was added to a suspension of sulfuric diamide (146 mg, 1.5 mmol, Eq: 1.0) and (S)-3-fluoropyrrolidine hydrochloride (234 mg, 1.8 mmol, Eq: 1.2) in dioxane (1.3 ml). The reaction was stirred in a sealed tube at 115°C for 16 h 35 min, then concentrated in vacuo. The residue was diluted with MeOH and evaporated with silica gel to dryness and transferred to a column. Purification by flash chromatography (40 g silica, 0-8% MeOH/DCM) gave the title compound as a light yellow solid (193 mg, 75% yield). MS (ESI) m/z: 169.1 [M+H]⁺.

### (3R)-N-[2-cyano-4-fluoro-3-(3-methyl-4-oxo-quinazolin-6-yl)oxy-phenyl]-3-fluoro-pyrrolidine-1-sulfonamide (compound of formula (Ia))

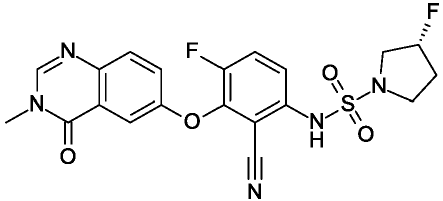

(*R*)-3-Fluoropyrrolidine-1-sulfonamide (1.26 g, 7.51 mmol, Eq: 2.1) and cesium carbonate (2.56 g, 7.87 mmol, Eq: 2.2) were suspended in dry DMF (10.2 ml) under an argon atmosphere. The reaction was stirred at 50 °C for 30 min. The reaction mixture was cooled to rt and a solution of 3,6-difluoro-2-((3-methyl-4-oxo-3,4-dihydroquinazolin-6-yl)oxy)benzonitrile (1.12 g, 3.58 mmol, Eq: 1.0) in DMF (25.5 ml) was added. The reaction mixture was stirred at 100 °C for 15 h, then concentrated in vacuo. The residue was taken up in sat. aq. NH₄Cl (100 mL) and EtOAc (100 mL). The phases were separated, and the aqueous layer was extracted further with 2 x 100 mL EtOAc. The combined organic layers were washed with water (200 mL) and brine (200 mL), dried (Na₂SO₄), filtered and concentrated in vacuo. The water layer was back-extracted with EtOAc (3 x 100 mL). The combined organic extracts were washed with brine (200 mL), dried (Na₂SO₄), filtered and concentrated in vacuo. The residue was diluted with DCM and MeOH, and concentrated onto silica. Purification by flash chromatography (120 g, 0.5-2% MeOH/DCM) gave an off-white solid which was triturated with 1:1 heptane/DCM (20 mL) with sonication, then dried in vacuo to give the title compound as a colourless solid (1.087 g, 66% yield). MS (ESI) m/z: 426.2 [M+H]⁺. Chiral SFC: RT = 4.594 min [Chiralpak IC column, 4.6 x 250 mm, 5 µm particle size (Daicel); gradient of 20 - 40% MeOH containing 0.2% NHEt₂ over 8 min; flow: 2.5 mL/min; 140 bar backpressure].

### (3S)-N-[2-cyano-4-fluoro-3-(3-methyl-4-oxo-quinazolin-6-yl)oxy-phenyl]-3-fluoro-pyrrolidine-1-sulfonamide (compound of formula (Ib))

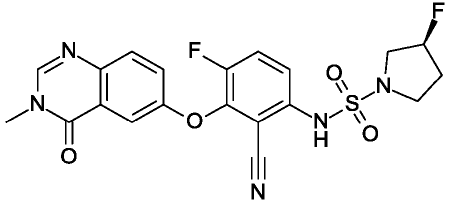

(*S*)-3-Fluoropyrrolidine-1-sulfonamide (181 mg, 1.08 mmol, Eq: 2.1) was dissolved in DMF (1.6 ml). At rt cesium carbonate (368 mg, 1.13 mmol, Eq: 2.2) was added and the reaction mixture was stirred at 50 °C for 30 min. The reaction mixture was cooled to rt and a solution of 3,6-difluoro-2-((3-methyl-4-oxo-3,4-dihydroquinazolin-6-yl)oxy)benzonitrile (160.8 mg, 513 µmol, Eq: 1.0) in DMF (4 ml) was added. The reaction mixture was stirred at 105 °C for 2 h 50 min then concentrated in vacuo. The residue was taken up in DCM and washed with sat. aq. NH₄Cl. The aq. layer was back-extracted twice with DCM. The combined organic layers were dried over Na₂SO₄, filtrated and evaporated. The residue (brown oil) was diluted with DCM and transferred to a column. Purification by flash chromatography (80 g, 0-100% EtOAc in DCM) gave a solid which was further purified by SFC to give the title compound as a light yellow solid (119 mg, 50% yield). MS (ESI) *m*/*z:* 426.2 [M+H]⁺. Chiral SFC: RT = 4.411 min [Chiralpak IC column, 4.6 x 250 mm, 5µm particle size (Daicel); gradient of 20 - 40% MeOH containing 0.2% NHEt₂ over 8 min; flow: 2.5 mL/min; 140 bar backpressure].

### 2-(4-cyclopropyl-2-fluoroanilino)-3,4-difluoro-5-[[3-fluoro-2-(methylsulfamoylamino)pyridin-4-yl]methyl]benzamide (Compound of formula (II))

The compound of formula (II) can be manufactured by the methods given below, by the methods given in the examples or by analogous methods.

NMR analysis was conducted using an AVANCE III HD400 (400 MHz) by Bruker Co. The NMR data were shown in ppm (parts per million) (δ) and the deuterium lock signal from the sample solvent was used as a reference.

The mass spectrum data were obtained using an ultra-high performance liquid chromatography (Nexera UC)-equipped single quadrupole mass spectrometer (LCMS-2020) by Shimadzu Corp. or an Acquity ultra-high performance liquid chromatography (UPLCor UPLC I-Class)-equipped single quadrupole mass spectrometer (SQD or SQD2) by Waters Co.

High-performance liquid chromatography was carried out using one of the analysis conditions A to C listed in Table 2 below. In Table 2, "TFA" stands for trifluoroacetic acid, "FA" for formic acid.

**Table 2:**

| Analysis conditions | Apparatus | Column | Column temperature | Detection wavelength (PDA) |
|---|---|---|---|---|
| A | Nexera UC LCMS-2020 | Ascentis Express C18 | 35°C | 210-400 nm |
| | | 2.1 mm, I.D. x 50 mm L, 2.7 µm | | |
| B | Acquity SQD/SQD2 | Ascentis Express C18 | 35°C | 210-400 nm |
| | | 2.1 mm, I.D. x 50 mm L, 2.7 µm | | |
| C | Acquity SQD/SQD2 | Ascentis Express C18 | 35°C | 210-400 nm |
| | | 2.1 mm, I.D. x 50 mm L, 2.7 µm | | |

**Table 2 (cont.)**

| Analysis conditions | Mobile phase | Gradient | | Flow rate (mL/min) |
|---|---|---|---|---|
| | | Time after injection (min) | A/B | |
| A | A) 0.05% TFA/CH₃CN | 0-1.5 | 5/95 → 100/0 | 1 |
| | B) 0.05% TFA/H₂O | 1.5-2.0 | 100/0 | |
| B | A) 0.1% FA/CH₃CN | 0-1.0 | 40/60 → 100/0 | 1 |
| | B) 0.1% FA/H₂O | 1.0-1.4 | 100/0 | |
| C | A) 0.1% FA/CH₃CN | 0-1.0 | 5/95 → 100/0 | 1 |
| | B) 0.1% FA/H₂O | 1.0-1.4 | 100/0 | |

Commercially available reagents were used directly without further purification.

All of the nonaqueous reactions were conducted in anhydrous solvents.

Concentration under reduced pressure and solvent distillation were carried out using a rotary evaporator.

As used herein, "room temperature" means a temperature of about 20°C to about 25°C.

### Methyl 3,4-difluoro-2-(2-fluoro-4-iodoanilino)-5-formylbenzoate (compound a1)

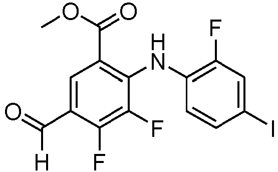

A mixed suspension of 3,4-difluoro-2-((2-fluoro-4-iodophenyl)amino)-5-formylbenzoic acid (5.50 g, 13.1 mmol) in toluene (44 mL) and MeOH (11 mL) was cooled to 0°C, a 10% diazomethyltrimethylsilane hexane solution (21.8 mL, 13.1 mmol) was added, and the mixture was stirred for 64 hours at room temperature. Acetic acid (0.748 mL) was added to the reaction mixture, which was then concentrated under reduced pressure. The resulting residue was purified by trituration (hexane/ethyl acetate) to give the title compound (5.01 g, 88%) as a colorless solid. LCMS m/z: 436 [M+H]⁺. HPLC retention time: 1.00 min (analysis conditions B)

### Methyl 3,4-difluoro-2-(2-fluoro-4-iodoanilino)-5-[(E)-[(4-methylphenyl)sulfonylhydrazinylidene]methyl]benzoate (compound a2)

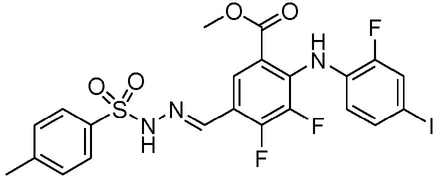

4-Methylbenzenesulfonyl hydrazide (2.14 g, 11.5 mmol) was added to a suspension of methyl 3,4-difluoro-2-(2-fluoro-4-iodoanilino)-5-formylbenzoate (compound a1, 5.00 g, 11.5 mmol) in EtOH (100 mL), and the mixture was stirred for 3 hours at room temperature. The reaction mixture was concentrated under reduced pressure, and then hexane (150 mL) was added.

The mixture was cooled to 0°C and filtered, and then washed with hexane (30 mL) to give the title compound (7.05 g, quant.) as a solid. LCMS m/z: 604 [M+H]⁺. HPLC retention time: 1.06 min (analysis conditions B)

### N-(2,4-Dimethoxybenzyl)-3-fluoro-4-iodopyridine-2-amine (compound a3)

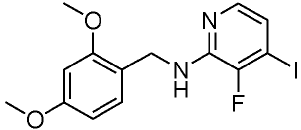

Triethylamine (3.63 mL, 26.0 mmol) and 1-(2,4-dimethoxyphenyl)methaneamine (3.26 mL, 21.7 mmol) were added to a solution of 2,3 -difluoro-4-iodopyridine (2.09 g, 8.67 mmol) in NMP (32 mL), and the mixture was stirred for 1.5 hours at 100°C. Water was added to the reaction mixture, and extraction was performed with ethyl acetate. The organic layer was washed with 13% brine, dried over anhydrous sodium sulfate and, after filtering off the drying agent, concentrated under reduced pressure. The resulting residue was purified by silica gel column chromatography (hexane/ ethyl acetate) to give the title compound (3.20 g, 95%) as an oil. LCMS m/z: 389 [M+H]⁺. HPLC retention time: 0.94 min (analysis conditions C)

### [2-[(2,4-Dimethoxyphenyl)methylamino]-3-fluoropyridin-4-yl]boronic acid (compound a4)

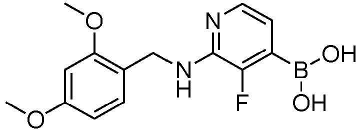

A 1,4-dioxane solution (27 mL) of N-(2,4-dimethoxybenzyl)-3-fluoro-4-iodopyridine-2-amine (compound a3, 2.70 g, 6.96 mmol), [1,1-bis(diphenylphosphino)ferrocene]dichloropalladium(II) dichloromethane addition product (568 mg, 0.696 mmol), potassium acetate (2.05 g, 20.9 mmol) and bis(pinacolato)diboron (2.65 g, 10.4 mmol) was stirred under a nitrogen atmosphere for 5 hours at 90°C and then for 19 hours at 110°C. The reaction mixture was concentrated under reduced pressure, and the resulting residue was purified by reversed-phase column chromatography (0.1% formic acid aqueous solution/ 0.1% formic acid acetonitrile solution) to give the title compound (2.07 g, 97%) as an oil. LCMS m/z: 307 [M+H]⁺. HPLC retention time: 0.44 min (analysis conditions C)

### Methyl 5-[[2-[(2,4-dimethoxyphenyl)methylamino]-3-fluoropyridin-4-yl]methyl]-3,4-difluoro-2-(2-fluoro-4-iodoanilino)benzoate (compound a5)

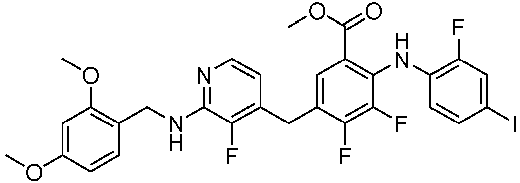

A 1,4-dioxane suspension (59 mL) of methyl 3,4-difluoro-2-(2-fluoro-4-iodoanilino)-5-[(E)-[(4-methylphenyl)sulfonylhydrazinylidene]methyl]benzoate (compound a2, 1.30 g, 2.16 mmol), [2-[(2,4-dimethoxyphenyl)methylamino]-3-fluoropyridin-4-yl]boronic acid (compound a4, 1.98 g, 6.46 mmol) and potassium carbonate (357 mg, 2.59 mmol) was stirred under a nitrogen atmosphere for 2.5 hours at 100°C and then for 3 hours at 110°C. Ethyl acetate was added to the reaction mixture, which was then washed with water and 13% brine. The organic layer was dried over anhydrous sodium sulfate and, after filtering off the drying agent, concentrated under reduced pressure. The resulting residue was purified by silica gel column chromatography (hexane/ ethyl acetate) to give the title compound (524 mg, 36%) as a foam. LCMS m/z: 682 [M+H]⁺. HPLC retention time: 1.03 min (analysis conditions B)

### Methyl 5-[(2-amino-3-fluoropyridin-4-yl)methyl]-3,4-difluoro-2-(2-fluoro-4-iodoanilino)benzoate (compound a6)

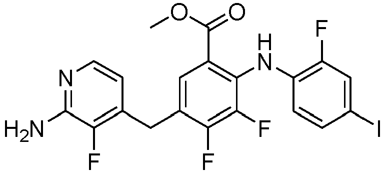

A DCM solution (16 mL) of methyl 5-[[2-[(2,4-dimethoxyphenyl)methylamino]-3-fluoropyridin-4-yl]methyl]-3,4-difluoro-2-(2-fluoro-4-iodoanilino)benzoate (compound a5, 523 mg, 0.768 mmol) was cooled to 0°C, trifluoroacetic acid (15.7 mL) was added, and the mixture was stirred for 1 hour at room temperature. The reaction mixture was concentrated under reduced pressure, and the resulting residue was purified by reversed-phase column chromatography (0.05% trifluoroacetic acid aqueous solution/ 0.05% trifluoroacetic acid acetonitrile solution) to give the title compound (321 mg, 79%) as an oil. LCMS m/z: 532 [M+H]⁺. HPLC retention time: 0.55 min (analysis conditions B)

### 5-((2-Amino-3-fluoropyridin-4-yl)methyl)-3,4-difluoro-2-((2-fluoro-4-iodophenyl)amino)benzoic acid hydrochloride (compound a7)

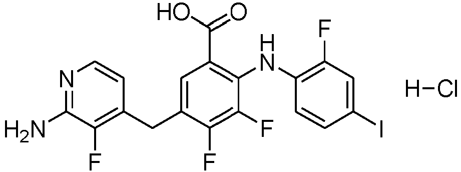

A mixed solution of methyl 5-[(2-amino-3-fluoropyridin-4-yl)methyl]-3,4-difluoro-2-(2-fluoro-4-iodoanilino)benzoate (compound a6, 4.00 g, 7.53 mmol) in THF (64 mL) and water (32 mL) was cooled to 0°C, lithium hydroxide monohydrate (948 mg, 22.6 mmol) was added, and the mixture was stirred for 3.5 hours at room temperature. After cooling to 0°C, 5 M hydrochloric acid (15.1 mL) was added to the reaction mixture, which was then concentrated under reduced pressure. The resulting residue was washed with water and TBME to give the title compound (4.20 g, quant.) as a violet compound. LCMS m/z: 518 [M+H]⁺. HPLC retention time: 0.68 min (analysis conditions C)

### 5-((2-Amino-3-fluoropyridin-4-yl)methyl)-3,4-difluoro-2-((2-fluoro-4-iodophenyl)amino)benzamide (compound a8)

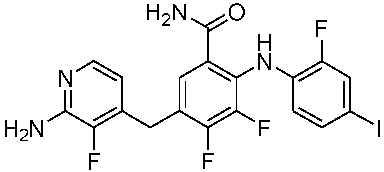

An anhydrous DMF solution (3.6 mL) of 5-((2-amino-3-fluoropyridin-4-yl)methyl)-3,4-difluoro-2-((2-fluoro-4-iodophenyl)amino)benzoic acid hydrochloride (compound a7, 200 mg, 0.361 mmol) was cooled to 0°C, HOOBt (67.8 mg, 0.415 mmol) and EDC·HCl (80.0 mg, 0.415 mmol) were added, and the mixture was stirred for 1.5 hours at room temperature. After further adding HOOBt (8.8 mg, 0.054 mmol) and EDC·HCl (10.4 mg, 0.054 mmol) and stirring at room temperature for 1 hour, a 7 M ammonia MeOH solution (0.103 mL, 0.722 mmol) and DIPEA (0.189 mL, 1.08 mmol) were added at 0°C and the mixture was stirred for 30 minutes at room temperature. Water and a saturated sodium hydrogen carbonate aqueous solution were added at 1:1 to the reaction mixture, and extraction was performed with ethyl acetate. The organic layer was dried over anhydrous sodium sulfate and, after filtering off the drying agent, concentrated under reduced pressure. The resulting residue was dissolved in ethyl acetate (1 mL), and hexane (10 mL) was added. The obtained solid was filtered and washed with hexane to give the title compound (162 mg, 87%) as a colorless solid. LCMS m/z: 517 [M+H]⁺. HPLC retention time: 0.64 min (analysis conditions C)

### 5-((2-Amino-3-fluoropyridin-4-yl)methyl)-2-((4-cyclopropyl-2-fluorophenyl)amino)-3,4-difluorobenzamide (compound a9)

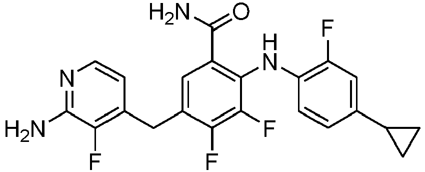

Tetrakis(triphenylphosphine)palladium(0) (11.2 mg, 9.68 µmol) and 0.5 M cyclopropylzinc bromide (1.94 mL, 0.969 mmol) were added to an anhydrous THF solution (1.9 mL) of 5-((2-amino-3-fluoropyridin-4-yl)methyl)-3,4-difluoro-2-((2-fluoro-4-iodophenyl)amino)benzamide (compound a8, 100 mg, 0.194 mmol), and the mixture was stirred for 2.5 hours at room temperature under a nitrogen atmosphere. Ethyl acetate (5 mL) was added to the reaction mixture, which was then filtered with Celite and washed with ethyl acetate (3 mL). The filtrate was washed with water and saturated brine, and the organic layer was dried over anhydrous sodium sulfate and, after filtering off the drying agent, concentrated under reduced pressure. Dichloromethane/hexane (1/10, 11 mL) was added to the resulting residue, and the solid was filtered off and washed with hexane (3 mL) to give the title compound a9 (63.4 mg, 76%) as a colorless solid. LCMS m/z: 431 [M+H]⁺. HPLC retention time: 0.61 min (analysis conditions C)

### 4-Nitrophenyl methylsulfamate (compound r1)

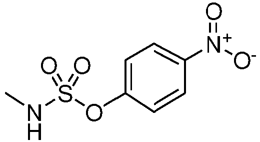

A dichloromethane solution (60 mL) of 4-nitrophenol (5.00 g, 35.9 mmol) and triethylamine (11.3 mL, 81.0 mmol) was cooled to -78°C, a dichloromethane solution (15 mL) of methylsulfamoyl chloride (5.82 g, 44.9 mmol) was added, and the mixture was stirred for 1.5 hours at -78°C. The reaction mixture was concentrated under reduced pressure, and the resulting residue was purified by silica gel column chromatography (hexane/ ethyl acetate) and reversed-phase column chromatography (0.1% formic acid aqueous solution/ 0.1% formic acid acetonitrile solution) to give the title compound (5.51 g, 66%) as a colorless solid. HPLC retention time: 0.63 min (analysis conditions C). ¹H-NMR (400 MHz, CDCl₃) δ: 8.31 (2H, m), 7.46 (2H, m), 4.68 (1H, m), 3.00 (3H, d, J = 5.4 Hz).

### 2-(4-Cyclopropyl-2-fluoroanilino)-3,4-difluoro-5-[[3-fluoro-2-(methylsulfamoylamino)pyridin-4-yl]methyl]benzamide (compound of formula (II))

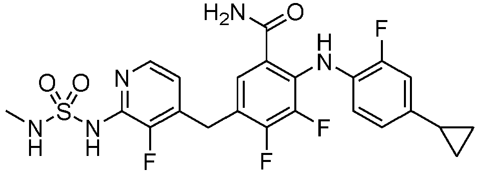

After dissolving 5 -((2-amino-3 -fluoropyridin-4-yl)methyl)-2-((4-cyclopropyl-2-fluorophenyl)amino)-3,4-difluorobenzamide (compound a9, 2.47 g, 5.74 mmol) in anhydrous DMF (28.7 mL), pyridine (2.78 mL, 34.4 mmol) and 4-nitrophenyl methylsulfamate (compound r1, 4.00 g, 17.2 mmol) were added and the mixture was stirred for 2.5 hours at 40°C. The reaction mixture was cooled to room temperature, and water (24.7 mL) was added. After further adding acetonitrile (3 mL) and water (19.8 mL) and stirring for 10 minutes, the solid was filtered out. The obtained solid was washed with water/acetonitrile (1/1, 49.4 mL) to give the title compound (2.56 g, 85%) as a colorless solid. LCMS m/z: 524 [M+H]⁺. HPLC retention time: 1.13 min (analysis conditions A).

### 2-(4-Cyclopropyl-2-fluoroanilino)-3,4-difluoro-5-[[3-fluoro-2-(methylsulfamoylamino)pyridin-4-yl]methyl]benzamide sodium salt (compound IIa)

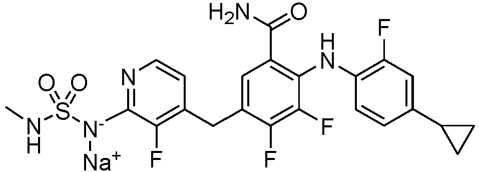

### (1) Preparation of compound IIa (Form I)

Acetone (10.6 mL) and DMSO (1.51 mL) were added to 2-(4-cyclopropyl-2-fluoroanilino)-3,4-difluoro-5-[[3-fluoro-2-(methylsulfamoylamino)pyridin-4-yl]methyl]benzamide (compound of formula (II), 3.03 g), dissolving it at room temperature. A 20% sodium ethoxide ethanol solution (3.03 mL) and seed crystals of a sodium salt of compound II (sample IIb mentioned below) were added to the solution and the mixture was stirred for 1 hour at room temperature, after which ethanol (15.1 mL) was added and the mixture was stirred at room temperature for 4 hours. Ethanol (15.1 mL) was then added, and the mixture was stirred for 4 hours at room temperature to give a sodium salt of compound II (2.74 g) as powdered crystals (sample IIa (Form I)).

### (2) Preparation of sample IIb

A 20% sodium ethoxide ethanol solution (0.054 mL) and methyl isobutyl ketone (0.161 mL) were added to the compound of formula (II) (53.6 mg), the mixture was stirred for 30 minutes at room temperature, and then methyl isobutyl ketone (0.161 mL) was added and stirring was continued for 4 days at 60°C. DMSO (0.054 mL) was then added, and the mixture was stirred for 5 hours at 60°C to give a sodium salt of compound II (25.6 mg) as powdered crystals (sample IIb).

### MEK1-inhibiting activity of Compound II

The MEK1-inhibiting activity of the compound II was evaluated by the fluorescent polarization method as described below.

The test compound, CRAF (Thermo Fisher Scientific Inc.), MEK1 (Thermo Fisher Scientific Inc.) and ERK2 (Carna Biosciences, Inc.) were mixed in ATP-containing buffer and reacted for 60 minutes at 30°C. FAM-labeled ERKtide (Molecular Devices Corp.) was then added and reaction was continued for 45 minutes at 30°C. IMAP (registered trademark) Progressive Binding Reagent (Molecular Devices Corp.) was further added, and reaction was continued for 15 minutes at room temperature. Following the reaction, the fluorescent polarization was measured with a fluorescent plate reader and the 50% inhibition concentration (IC50) was calculated based on the percent inhibition relative to a test compound-free control. An IC50 of 17 nM for MEK1 activity was measured when the test compound was compound II.
The invention relates in particular to:
A combination of a BRAF inhibitor and a MEK inhibitor wherein the BRAF inhibitor is a compound of formula (I) 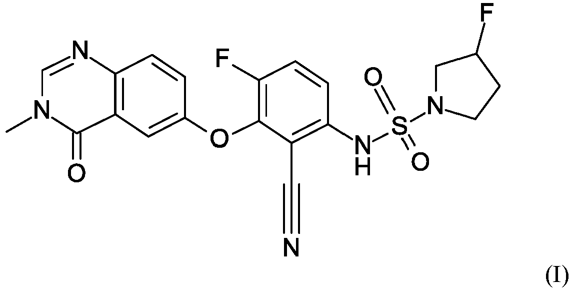 or a pharmaceutically acceptable salt or solvate thereof, wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or pharmaceutically acceptable salts thereof;
A combination of a BRAF inhibitor and a MEK inhibitor according to the invention, wherein the compound of formula (I) is (3R)-N-[2-cyano-4-fluoro-3-(3-methyl-4-oxo-quinazolin-6-yl)oxy-phenyl]-3-fluoro-pyrrolidine-1-sulfonamide;
A combination of a BRAF inhibitor and a MEK inhibitor according to the invention, wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or pharmaceutically acceptable salts thereof;
A combination of a BRAF inhibitor and a MEK inhibitor according to the invention, wherein the MEK inhibitor is 2-(4-cyclopropyl-2-fluoroanilino)-3,4-difluoro-5-[[3-fluoro-2-(methylsulfamoylamino)pyridin-4-yl]methyl]benzamide or a pharmaceutically acceptable salt thereof;
A combination of a BRAF inhibitor and a MEK inhibitor according to the invention, wherein the MEK inhibitor is 2-(4-cyclopropyl-2-fluoroanilino)-3,4-difluoro-5-[[3-fluoro-2-(methylsulfamoylamino)pyridin-4-yl]methyl]benzamide sodium salt;
A combination of a BRAF inhibitor and a MEK inhibitor according to the invention, wherein the MEK inhibitor is cobimetinib or a pharmaceutically acceptable salt thereof;
A combination of a BRAF inhibitor and a MEK inhibitor according to the invention, wherein the MEK inhibitor is [3,4-difluoro-2-(2-fluoro-4-iodoanilino)phenyl]-[3-hydroxy-3-[(2S)-piperidin-2-yl]azetidin-1-yl]methanone hemifumarate;
A combination of a BRAF inhibitor and a MEK inhibitor according to the invention, wherein the MEK inhibitor is [3,4-difluoro-2-(2-fluoro-4-iodoanilino)phenyl]-[3-hydroxy-3-[(2S)-piperidin-2-yl]azetidin-1-yl]methanone hemisuccinate;
A combination of a BRAF inhibitor and a MEK inhibitor according to the invention, for use as a medicament;
A combination of a BRAF inhibitor and a MEK inhibitor according to the invention, for use in the therapeutic and/or prophylactic treatment of cancer;
A pharmaceutical composition comprising a combination of a BRAF inhibitor and a MEK inhibitor according to the invention, and one or more pharmaceutically acceptable excipients;
A combination or a pharmaceutical composition as described herein, wherein the BRAF inhibitor and the MEK inhibitor are both administered orally;
A combination or a pharmaceutical composition as described herein, wherein the BRAF inhibitor is administered concurrently with the MEK inhibitor;
A combination or a pharmaceutical composition according to the invention, wherein the BRAF inhibitor and the MEK inhibitor are co-formulated;
A combination or a pharmaceutical composition according to the invention, wherein the BRAF inhibitor is administered sequentially with the MEK inhibitor;
A combination of a BRAF inhibitor and a MEK inhibitor for use according to the invention, wherein the cancer is thyroid cancer, colorectal cancer, melanoma, brain cancer or non-small cell lung cancer;
A combination of a BRAF inhibitor and a MEK inhibitor for use according to the invention, wherein the cancer is associated with BRAF^{V600} mutations;
A combination of a BRAF inhibitor and a MEK inhibitor for use according to the invention, wherein the cancer is BRAF^{V600} mutation-positive unresectable or metastatic cancer;
A combination BRAF inhibitor and a MEK inhibitor for use as described herein, wherein BRAF^{V600} mutation is determined using a method comprising (a) performing PCR or sequencing on nucleic acid (e.g., DNA) extracted from a sample of the patient's tumour tissue and/or body fluid; and (b) determining expression of BRAF^{V600} in the sample;
A combination of a BRAF inhibitor and a MEK inhibitor for use according to the invention, comprising one or more additional anticancer agents selected from MEK degraders, EGFR inhibitors, EGFR degraders, inhibitors of HER2 and/or HER3, degraders of HER2 and/or HER3, SHP2 inhibitors, SHP2 degraders, Axl inhibitors, Axl degraders, ALK inhibitors, ALK degraders, PI3K inhibitors, PI3K degraders, SOS1 inhibitors, SOS1 degraders, signal transduction patway inhibitors, checkpoint inhibitors, modulators of the apoptosis pathway, cytotoxic chemotherapeutics, angiogenesis-targeted therapies, immune-targeted agents, and antibody-drug conjugates;
A pharmaceutical composition as described herein, wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or from a pharmaceutically acceptable salt thereof;
A pharmaceutical composition as described herein, wherein the MEK inhibitor is 2-(4-cyclopropyl-2-fluoroanilino)-3,4-difluoro-5-[[3-fluoro-2-(methylsulfamoylamino)pyridin-4-yl]methyl]benzamide or a pharmaceutically acceptable salt thereof;
A pharmaceutical composition as described herein, wherein the MEK inhibitor is cobimetinib or a pharmaceutically acceptable salt thereof;
A pharmaceutical composition as described herein, wherein the compound of formula (I) is (3R)-N-[2-cyano-4-fluoro-3-(3-methyl-4-oxo-quinazolin-6-yl)oxy-phenyl]-3-fluoro-pyrrolidine-1-sulfonamide;
A pharmaceutical composition as described herein, wherein the compound of formula (I) is (3S)-N-[2-cyano-4-fluoro-3-(3-methyl-4-oxo-quinazolin-6-yl)oxy-phenyl]-3-fluoro-pyrrolidine-1-sulfonamide;
A pharmaceutical composition as described herein, for use in the treatment or prophylaxis of cancer, in particular thyroid cancer, colorectal cancer, melanoma, brain cancer or non-small cell lung cancer;
A pharmaceutical composition as described herein, for use in the treatment or prophylaxis of cancer, in particular thyroid cancer, colorectal cancer, melanoma, brain cancer or non-small cell lung cancer, wherein the BRAF inhibitor and the MEK inhibitor are administered orally;
A pharmaceutical composition as described herein, for use in the treatment or prophylaxis of cancer, in particular thyroid cancer, colorectal cancer, melanoma, brain cancer or non-small cell lung cancer, wherein the first composition is administered concurrently with the second composition;
A pharmaceutical composition as described herein, for use in the treatment or prophylaxis of cancer, in particular thyroid cancer, colorectal cancer, melanoma, brain cancer or non-small cell lung cancer, wherein the first and second compositions are co-formulated;
A pharmaceutical composition as described herein, for use in the treatment or prophylaxis of cancer, in particular thyroid cancer, colorectal cancer, melanoma, brain cancer or non-small cell lung cancer, wherein the first composition is administered sequentially with the second composition;
A pharmaceutical composition as described herein, for use in the treatment or prophylaxis of cancer, in particular thyroid cancer, colorectal cancer, melanoma, brain cancer or non-small cell lung cancer;
A pharmaceutical composition as described herein, for use in the treatment or prophylaxis of cancer, wherein the cancer is associated to BRAF mutations;
A pharmaceutical composition as described herein, for use in the treatment or prophylaxis of cancer, wherein the cancer is BRAF^{V600} mutation-positive unresectable or metastatic cancer, in particular BRAF^{V600E} or BRAF^{V600K} mutation-positive unresectable or metastatic cancer; and
A pharmaceutical composition as described herein, for use in the treatment or prophylaxis of cancer, in particular thyroid cancer, colorectal cancer, melanoma, brain cancer or non-small cell lung cancer, wherein BRAF^{V600} mutation is determined using a method comprising (a) performing PCR or sequencing on nucleic acid (e.g., DNA) extracted from a sample of the patient's tumour tissue and/or body fluid; and (b) determining expression of BRAF^{V600} in the sample.

Further embodiments of the invention are:
[1] A compound for use in the treatment or prophylaxis of cancer in combination with a MEK inhibitor, wherein the compound is a compound of formula (I) 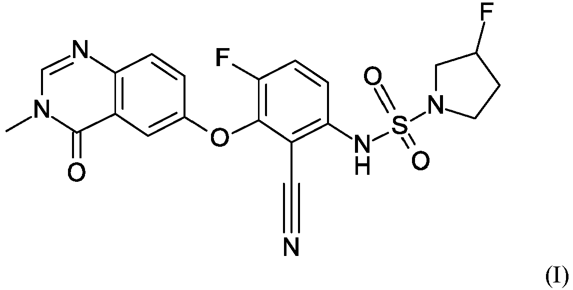 or a pharmaceutically acceptable salt or solvate thereof; wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or pharmaceutically acceptable salts thereof.
[2] A pharmaceutical composition comprising the compound according to [1] and one or more pharmaceutically acceptable excipients for the treatment or prophylaxis of cancer in combination with a MEK inhibitor wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or pharmaceutically acceptable salts thereof.
[3] The compound or the pharmaceutical composition according to any one of [1] to [2], wherein the compound of formula (I) is (3R)-N-[2-cyano-4-fluoro-3-(3-methyl-4-oxo-quinazolin-6-yl)oxy-phenyl]-3-fluoro-pyrrolidine-1-sulfonamide.
[4] The compound or the pharmaceutical composition according to any one of [1] to [3], wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or from a pharmaceutically acceptable salt or solvate thereof.
[5] The compound or the pharmaceutical composition according to any one of [1] to [4], wherein the MEK inhibitor is 2-(4-cyclopropyl-2-fluoroanilino)-3,4-difluoro-5-[[3-fluoro-2-(methylsulfamoylamino)pyridin-4-yl]methyl]benzamide or a pharmaceutically acceptable salt or solvate thereof.
[6.1] The compound or the pharmaceutical composition according to any one of [1] to [5], wherein the MEK inhibitor is 2-(4-cyclopropyl-2-fluoroanilino)-3,4-difluoro-5-[[3-fluoro-2-(methylsulfamoylamino)pyridin-4-yl]methyl]benzamide or a pharmaceutically acceptable salt thereof.
[7] The compound or the pharmaceutical composition according to any one of [1] to [6], wherein the MEK inhibitor is 2-(4-cyclopropyl-2-fluoroanilino)-3,4-difluoro-5-[[3-fluoro-2-(methylsulfamoylamino)pyridin-4-yl]methyl]benzamide sodium salt.
[8] The compound or the pharmaceutical composition according to any one of [1] to [4], wherein the MEK inhibitor is cobimetinib or a pharmaceutically acceptable salt or solvate thereof.
[9] The compound or the pharmaceutical composition according to any one of [1] to [8], wherein the compound of formula (I) and the MEK inhibitor are both administered orally.
[10] The compound or the pharmaceutical composition according to any one of [1] to [9], wherein the compound of formula (I) is administered concurrently with the MEK inhibitor.
[11] The compound or the pharmaceutical composition according to any one of [1] to [10], wherein the compound of formula (I) is administered sequentially with the MEK inhibitor.
[12] The compound or the pharmaceutical composition according to any one of [1] to [11], wherein the cancer is thyroid cancer, colorectal cancer, melanoma, brain cancer or non-small cell lung cancer.
[13] The compound or the pharmaceutical composition according to any one of [1] to [12], wherein the cancer is associated with BRAFV600 mutations.
[14] The compound or the pharmaceutical composition according to any one of [1] to [13], wherein the cancer is BRAFV600 mutation-positive unresectable or metastatic cancer.
[15] The compound or the pharmaceutical composition according to any one of [1] to [14], wherein BRAFV600 mutation is determined using a method comprising (a) performing PCR or sequencing on nucleic acid (e.g., DNA) extracted from a sample of the patient's tumour tissue and/or body fluid; and (b) determining expression of BRAFV600 in the sample.
[16] The compound or the pharmaceutical composition according to any one of [1] to [15], comprising one or more additional anticancer agents selected from MEK degraders, EGFR inhibitors, EGFR degraders, inhibitors of HER2 and/or HER3, degraders of HER2 and/or HER3, SHP2 inhibitors, SHP2 degraders, Axl inhibitors, Axl degraders, ALK inhibitors, ALK degraders, PI3K inhibitors, PI3K degraders, SOS1 inhibitors, SOS1 degraders, signal transduction patway inhibitors, checkpoint inhibitors, modulators of the apoptosis pathway, cytotoxic chemotherapeutics, angiogenesis-targeted therapies, immune-targeted agents, and antibody-drug conjugates.

A certain embodiment of the invention relates to a pharmaceutical composition as described herein, for the use as a medicament in therapeutic and/or prophylactic treatment of brain metastases;
A certain embodiment of the invention relates to a pharmaceutical composition as described herein, for the use as a medicament in therapeutic and/or prophylactic treatment of brain metastases, wherein the primary tumour is melanoma or non-small cell lung cancer;
A certain embodiment of the invention relates to a pharmaceutical composition as described herein, for use in the treatment and/or prophylaxis cancer, in particular melanoma or non-small cell lung cancer, wherein the patient is treatment-naive regading targeted therapy;
A certain embodiment of the invention relates to a pharmaceutical composition as described herein, for use in the treatment and/or prophylaxis cancer, in particular melanoma or non-small cell lung cancer, wherein the patient is treatment-naive regading targeted therapy, and wherein the patient is checkpoint inhibitor treatment-experienced;
A certain embodiment of the invention relates to a pharmaceutical composition as described herein, for use in the treatment and/or prophylaxis cancer, in particular melanoma or non-small cell lung cancer, wherein the patient is treatment-experienced regarding targeted therapy, and wherein the patient is checkpoint inhibitor treatment-experienced;
A certain embodiment of the invention relates to a pharmaceutical composition as described herein, for use in the treatment and/or prophylaxis cancer, in particular melanoma or non-small cell lung cancer, wherein the cancer was previously treated by surgery;
A certain embodiment of the invention relates to a pharmaceutical composition as described herein, for use in the treatment and/or prophylaxis of cancer, in particular melanoma or non-small cell lung cancer, wherein the patient is treatment-naive regarding BRAF inhibitor treatment;
A certain embodiment of the invention relates to a pharmaceutical composition as described herein, for the use as a medicament in therapeutic and/or prophylactic treatment of BRAF inhibitor treatment-resistant tumour;
A certain embodiment of the invention relates to a pharmaceutical composition as described herein, for use in the treatment and/or prophylaxis of cancer, in particular melanoma or non-small cell lung cancer, wherein the patient is treatment-naive regarding MEK inhibitor treatment; and
A certain embodiment of the invention relates to a pharmaceutical composition as described herein, for the use as a medicament in therapeutic and/or prophylactic treatment of MEK inhibitor treatment-resistant tumour.
A certain embodiment of the invention relates to a pharmaceutical composition as described herein, for the use as a medicament in therapeutic and/or prophylactic treatment of cancer in a subject which was previously treated with a BRAF inhibitor selected from encorafenib, dabrafenib and encorafenib, and/or a MEK inhibitor selected from binimetinib, trametinib and cobimetinib;
A certain embodiment of the invention relates to a pharmaceutical composition as described herein, for the use as a medicament in therapeutic and/or prophylactic treatment in a subject which was previously treated with encorafenib and binimetinib;
A certain embodiment of the invention relates to a pharmaceutical composition as described herein, for the use as a medicament in therapeutic and/or prophylactic treatment in a subject which was previously treated with dabarafenib and trametinib;
A certain embodiment of the invention relates to a pharmaceutical composition as described herein, for the use as a medicament in therapeutic and/or prophylactic treatment in a subject which was previously treated with vemurafenib and cobimetinib;
A certain embodiment of the invention relates to a pharmaceutical composition as described herein, for the use as a medicament in therapeutic and/or prophylactic treatment of cancer, in particular melanoma or non-small cell lung cancer, in a subject which was previously treated with a checkpoint inhibitor;
A certain embodiment of the invention relates to a BRAF inhibitor and a MEK inhibitor as described herein, for use in the therapeutic and/or prophylactic treatment of cancer, in particular melanoma or non-small cell lung cancer, wherein the patient is treatment-naive regarding targeted therapy;
A certain embodiment of the invention relates to a BRAF inhibitor and a MEK inhibitor as described herein, for use in the therapeutic and/or prophylactic treatment of cancer, in particular melanoma or non-small cell lung cancer, wherein the patient is treatment-naive regarding targeted therapy, and wherein the patient is checkpoint inhibitor treatment-experienced;
A certain embodiment of the invention relates to a BRAF inhibitor and a MEK inhibitor as described herein, for use in the therapeutic and/or prophylactic treatment of cancer, wherein the patient is treatment-experienced regarding targeted therapy, and wherein the patient is checkpoint inhibitor treatment-experienced;
A certain embodiment of the invention relates to a BRAF inhibitor and a MEK inhibitor as described herein, for use in the therapeutic and/or prophylactic treatment of cancer, in particular melanoma or non-small cell lung cancer, wherein the patient is treatment-naive regarding BRAF inhibitor treatment;
A certain embodiment of the invention relates to a BRAF inhibitor and a MEK inhibitor as described herein, for the use as a medicament in therapeutic and/or prophylactic treatment of BRAF inhibitor treatment-resistant tumour;
A certain embodiment of the invention relates to a BRAF inhibitor and a MEK inhibitor as described herein, for use in the therapeutic and/or prophylactic treatment of cancer, in particular melanoma or non-small cell lung cancer, wherein the patient is treatment-naive regarding MEK inhibitor treatment;
A certain embodiment of the invention relates to a BRAF inhibitor and a MEK inhibitor as described herein, for use in therapeutic and/or prophylactic treatment of MEK inhibitor treatment-resistant tumour;
A certain embodiment of the invention relates to a BRAF inhibitor and a MEK inhibitor as described herein, for the use as a medicament in therapeutic and/or prophylactic treatment of cancer in a subject which was previously treated with a BRAF inhibitor selected from encorafenib, dabrafenib and encorafenib, and/or a MEK inhibitor selected from binimetinib, trametinib and cobimetinib;
A certain embodiment of the invention relates to a BRAF inhibitor and a MEK inhibitor as described herein, for the use as a medicament in therapeutic and/or prophylactic treatment in a subject which was previously treated with encorafenib and binimetinib;
A certain embodiment of the invention relates to a BRAF inhibitor and a MEK inhibitor as described herein, for the use as a medicament in therapeutic and/or prophylactic treatment in a subject which was previously treated with dabarafenib and trametinib;
A certain embodiment of the invention relates to a BRAF inhibitor and a MEK inhibitor as described herein, for the use as a medicament in therapeutic and/or prophylactic treatment in a subject which was previously treated with vemurafenib and cobimetinib;
A certain embodiment of the invention relates to a BRAF inhibitor and a MEK inhibitor as described herein, for use in therapeutic and/or prophylactic treatment of cancer, in particular melanoma or non-small cell lung cancer, in a subject which was previously treated with a checkpoint inhibitor;
In one embodiment the invention provides a kit comprising a BRAF inhibitor and a MEK inhibitor as as described therein, prescribing information also known as "leaflet", a blister package or bottle (HDPE or glass) and a container. The prescribing information preferably includes the advice to a patient regarding the administration of the combination of the BRAF inhibitor and the MEK inhibitor as described herein;
In one embodiment, the treated subject became refractory to said prior treatment as described herein; and
In one embodiment, the treated subject developed brain metastasis during said prior treatment as described herein.

A certain embodiment of the invention relates to a pharmaceutical composition comprising a compound of formula (I) as described herein, or a pharmaceutically acceptable salt or solvate thereof, wherein at least one substituent comprises at least one radioisotope. Particular examples of radioisotopes are ²H, ³H, ¹³C, ¹⁴C and ¹⁸F.

Furthermore, the invention includes all optical isomers, i.e. diastereoisomers, diastereomeric mixtures, racemic mixtures, all their corresponding enantiomers and/or tautomers as well as their solvates, wherever applicable, of the compound of formula (I).

Furthermore, the invention includes all optical isomers, i.e. diastereoisomers, diastereomeric mixtures, racemic mixtures, all their corresponding enantiomers and/or tautomers as well as their solvates, wherever applicable, of the MEK inhibitor.

If desired, racemic mixtures of the compound of the invention may be separated so that the individual enantiomers are isolated. The separation can be carried out by methods well known in the art, such as the coupling of a racemic mixture of compounds to an enantiomerically pure compound to form a diastereomeric mixture, followed by separation of the individual diastereomers by standard methods, such as fractional crystallization or chromatography.

In the embodiments, where an optically pure enantiomer is provided, optically pure enantiomer means that the compound contains > 90 % of the desired isomer by weight, particularly > 95 % of the desired isomer by weight, or more particularly > 99 % of the desired isomer by weight, said weight percent based upon the total weight of the isomer of the compound. A chirally pure or chirally enriched compound may be prepared by chirally selective synthesis or by separation of enantiomers. The separation of enantiomers may be carried out on the final product or alternatively on a suitable intermediate.

In one embodiment, one or more additional anticancer agents is used in combination with a BRAF inhibitor and a MEK inhibitor as described herein, wherein the additional anticancer agents are selected from MEK degraders, EGFR inhibitors, EGFR degraders, inhibitors of HER2 and/or HER3, degraders of HER2 and/or HER3, SHP2 inhibitors, SHP2 degraders, Axl inhibitors, Axl degraders, ALK inhibitors, ALK degraders, PI3K inhibitors, PI3K degraders, SOS1 inhibitors, SOS1 degraders, signal transduction patway inhibitors, checkpoint inhibitors, modulators of the apoptosis pathway, cytotoxic chemotherapeutics, angiogenesis-targeted therapies, immune-targeted agents, and antibody-drug conjugates.

In some embodiments, one of the additional anticancer agents is an EGFR inhibitor. Non-limiting examples of EGFR inhibitors include cetuximab (Erbitux^{®}), panitumumab (Vectibix^{®}), osimertinib (merelectinib, Tagrisso^{®}), erlotinib (Tarceva^{®}), gefitinib (Iressa^{®}), necitumumab (PortrazzaTM), neratinib (Nerlynx^{®}), lapatinib (Tykerb^{®}), vandetanib (Caprelsa^{®}) and brigatinib (Alunbrig^{®}). Additional examples of EGFR inhibitors are known in the art. In some embodiments the EGFR inhibitor is an allosteric EGFR inhibitor.

In some embodiments, one of the additional anticancer agents is an inhibitor of HER2 and/or HER3. Non-limiting examples of HER2 and/or HER3 inhibitors include Iapatinib, canertinib, (E)-2-methoxy-N-(3-(4-(3-methyl-4-(6-methylpyridin-3-yloxy)phenylamino)quinazolin-6-yl)allyl)acetamide (GP-724714), sapitinib, 7-[[4-[(3-ethynylphenyl)amino]-7-methoxy-6-quinazolinyl]oxy]-N-hydroxy-heptanamide (CUDC-101), mubritinib, 6-[4-[(4-ethylpiperazin-1-yl)methyl]phenyl]-N-[(1R)-1-phenylethyl]-7H-pyrrolo[2,3-d]pyrimidin-4-amine (AEE788), irbinitinib (tucatinib), poziotinib, N-[4-[1-[4-(4-acetyl-1-piperazinyl)cyclohexyl]-4-amino-3-pyrazolo[3,4-d]pyrimidinyl]-2-methoxyphenyl]-1-methyl-2-indolecarboxamide (KIN001-111), 7-cyclopentyl-5-(4-phenoxyphenyl)-7H-pyrrolo[2,3-d]pyrimidin-4-ylamine (KIN001-051), 6,7-dimethoxy-N-(4-phenoxyphenyl)quinazolin-4-amine (KIN001-30), dasatinib, andbosutinib.

In some embodiments, one of the additional anticancer agents is an inhibitor of SHP2. Non-limiting examples of SHP2 inhibitors include 6-(4-amino-4-methylpiperidin-1-yl)-3-(2,3-dichlorophenyl)pyrazine-2-amine (SHP099), [3-[(3S,4S)-4-amino-3-methyl-2-oxa-8-azaspiro[4.5]decan-8-yl]-6-(2,3-dichlorophenyl)-5-methylpyrazin-2-yl]methanol (RMC-4550) RMC-4630, TNO155, and the compounds disclosed in WO 2015/107493, WO 2015/107494, WO 2015/107495, WO 2019/075265, PCT/U82019/056786 and PCT/182020/053019.

In some embodiments, one of the additional anticancer agents is a PI3K inhibitor. Non-limiting examples include buparlisib (BKM120), alpelisib (BYL719), samotolisib (LY3023414), 8-[(1R)-1-[(3,5-difluorophenyl)amino]ethyl]-N,N-dimethyl-2-(morpholin-4-yl)-4-oxo-4H-chromene-6-carboxamide (AZD8186), tenalisib (RP6530), voxtalisib hydrochloride (SAR-245409), gedatolisib (PF-05212384), panulisib (P-7170), taselisib (GDC-0032), trans-2-amino-8-[4-(2-hydroxyethoxy)cyclohexyl]-6-(6-methoxypyridin-3-yl)-4-methylpyrido[2,3-d]pyrimidin-7(8H)-one (PF-04691502), duvelisib (ABBV-954), N2-[4-oxo-4-[4-(4-oxo-8-phenyl-4H-1-benzopyran-2-yl)morpholin-4-ium-4-ylmethoxy]butyryl]-L-arginyl-glycyl-L-aspartyl-L-serine acetate (SF-1126), pictilisib (GDC-0941), 2-methyl-1-[2-methyl-3-(trifluoromethyl)benzyl]-6-(morpholin-4-yl)-1H-benzimidazole-4-carboxylic acid (GSK2636771), idelalisib (GS-1101), umbralisib tosylate (TGR-1202), pictilisib (GDC-0941), copanlisib hydrochloride (BAY 84-1236), dactolisib (BEZ-235), 1-(4-[5-[5-amino-6-(5-tert-butyl-1,3,4-oxadiazol-2-yl)pyrazin-2-yl]-1-ethyl-1-H-1,2,4-triazol-3-yl]piperidin-1-yl)-3-hydroxypropan-1-one (AZD-8835), 5-[6,6-dimethyl-4-(morpholin-4-yl)-8,9-dihydro-6H-[1,4]oxazino[4,3-e]purin-2-yl]pyrimidin-2-amine (GDC-0084) everolimus, rapamycin, perifosine, sirolimus and temsirolimus.

In some embodiments, one of the additional anticancer agents is an ALK inhibitor. Non-limiting examples include crizotinib (PF-02341066), ceritinib (LDK378), alectinib (alecensa), brigatinib (AP26113), lorlatinib (PF-6463922), ensartinib (X-396), entrectinib (RXDX-101), reprotectinib (TPX-0005), belizatinib (TSR-011), alkotinib (ZG-0418), foritinib (SAF-189), CEP-37440, TQ-B3139, PLB1003 and TPX-0131

In some embodiments, one of the additional anticancer agents is a checkpoint inhibitor. In some embodiments, the checkpoint inhibitor is a CTLA-4 inhibitor, a PD-1 inhibitor or a PD-L1 inhibitor. In some embodiments, the CTLA-4 inhibitor is ipilimumab (Yervoy^{®}) or tremelimumab (GP-675,206). In some embodiments, the PD-1 inhibitor is pembrolizumab (Keytruda^{®}), nivolumab (Opdivo^{®}) and RN888. In some embodiments, the PD-L1 inhibitor is atezolizumab (Tecentriq^{®}), avelumab (Bavencio^{®}) or durvalumab (Imfinzi^{™}).

In some embodiments, one of the additional anticancer agents is an antibody-drug conjugate. Non-limiting examples of an antibody-drug conjugate include gemtuzumab ozogamicin (MylotargTM), inotuzumab ozogamicin (Besponsa^{®}), brentuximab vedotin (Adcetris^{®}), adotrastuzumab emtansine (TDM-f; Kadcyla^{®}), mirvetuximab soravtansine (IMGN853) and anetumab ravtansine.

In some embodiments, one of the additional anticancer agents is an antibody such as bevacizumab (MvastiTM, Avastin^{®}), trastuzumab (Herceptin^{®}), avelumab (Bavencio^{®}), rituximab (MabTheraTM, Rituxan^{®}), edrecolomab (Panorex), daratumuab (Darzalex^{®}), olaratumab (LartruvoTM), ofatumumab (Arzerra^{®}), alemtuzumab (Campath^{®}), cetuximab (Erbitux^{®}), oregovomab, pembrolizumab (Keytruda^{®}), dinutiximab (Unituxin^{®}), obinutuzumab (Gazyva^{®}), tremelimumab (GP-675,206), ramucirumab (Cyramza^{®}), ublituximab (TG-1101), panitumumab (Vectibix^{®}), elotuzumab (EmplicitiT'V'), necitumumab (PortrazzaT'V'), cirmtuzumab (UC-961), ibritumomab (Zevalin^{®}), isatuximab (SAR650984), nimotuzumab, fresolimumab (GC1008), Iirilumab (INN), mogamulizumab (Poteligeo^{®}), ficlatuzumab (AV-299), denosumab (Xgeva^{®}), ganitumab, urelumab, pidilizumab, amatuximab, blinatumomab (AMG103; Blincyto^{®}) or midostaurin (Rydapt).

Another embodiment of the invention provides a pharmaceutical composition containing one or more compositions, wherein each composition contains one or more compounds for use according to the invention and one or more therapeutically inert carriers, diluents or excipients, as well as a method to prepare such a pharmaceutical compositions. In one example, the compound of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compound of formula (I) is sterile. The compound may be stored, for example, as a solid or amorphous composition, as a lyophilized formulation or as an aqueous solution. In one example, the MEK inhibitor may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, the MEK inhibitor is formulated in an acetate buffer, at pH 5. In another embodiment, the MEK inhibitor is sterile. The MEK inhibitor may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

As used herein, a "pharmaceutically acceptable carrier" or "pharmaceutically acceptable excipient" is intended to include any and all material compatible with pharmaceutical administration including solvents, dispersion media, coatings, antibacterial and antifungal agents, isotonic and absorption delaying agents, and other materials and compounds compatible with pharmaceutical administration. Except insofar as any conventional media or agent is incompatible with the active compound, use thereof in the compositions of the invention is contemplated. Supplementary active compounds can also be incorporated into the compositions.

Pharmaceutical compositions can be obtained by processing the BRAF inhibitor as described herein and/or the MEK inhibitor with pharmaceutically acceptable, inorganic or organic carriers or excipients. Lactose, corn starch or derivatives thereof, talc, stearic acids or it's salts can be used, for example, as such carriers for tablets, coated tablets, dragées and hard gelatine capsules. Suitable carriers for soft gelatine capsules are, for example, vegetable oils, waxes, fats, semi-solid and liquid polyols. Depending on the nature of the active substance no carriers are, however, usually required in the case of soft gelatine capsules. Suitable carriers for the production of solutions and syrups are, for example, water, polyols, glycerol, vegetable oil. Suitable carriers for suppositories are, for example, natural or hardened oils, waxes, fats, semi-liquid or liquid polyols.

The pharmaceutical compositions can, moreover, contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Pharmaceutical compositions of a BRAF inhibitor and a MEK inhibitor, alone or in combination, can be prepared for storage by mixing the active ingredient having the desired degree of purity with optional pharmaceutically acceptable carriers, excipients or stabilizers (Remington's Pharmaceutical Sciences 16th edition, Osol, A. (ed.) (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEENTM, PLURONICSTM or polyethylene glycol (PEG).

Pharmaceutical compositions of a BRAF inhibitor and a MEK inhibitor include those suitable for oral, nasal, topical (including buccal and sublingual), rectal, vaginal and/or parenteral administration. The compositions may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of a BRAF inhibitor or a MEK inhibitor which produces a therapeutic effect. Generally, out of one hundred percent, this amount will range from about 1 percent to about 90 percent of active ingredient, preferably from about 5 percent to about 70 percent, most preferably from about 10 percent to about 30 percent. Methods of preparing these compositions include the step of bringing into association a BRAF inhibitor or a MEK inhibitor with the carrier and, optionally, one or more accessory ingredients. In general, the pharmaceutical compositions can be prepared by uniformaly and intimately bringing into association a BRAF inbitor and a MEK inhibitor with liquid carriers, or finely divided solid carriers, or both, and then, if necessary, shaping the product. Pharmaceutical compositions suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes, each containing a predetermined amount of a BRAF inhibitor and a MEK inhibitor as an active ingredient. A BRAF inhibitor and a MEK inhibitor may also be administered as a bolus, electuary or paste.

In further embodiments of the invention, a BRAF inhibitor and a MEK inhibitor are formulated into one or two separate pharmaceutical compositions.

The active ingredients may also be entrapped in microcapsules prepared, for example, by coacervation techniques or by interracial polymerization, for example, hydroxymethylcellulose or gelatin-microcapsules and poly- (methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences, 16th edition, Osol, A. (ed.) (1980).

The formulations to be used for *in vivo* administration must be sterile. This can be readily accomplished by filtration through sterile filtration membranes.

The dosage can vary within wide limits and will, of course, have to be adjusted to the individual requirements in each particular case. In the case of oral administration the dosage for adults can vary from about 0.01 mg to about 1000 mg per day of a compound of general formula (I) or of the corresponding amount of a pharmaceutically acceptable solvate thereof. The daily dosage may be administered as single dose or in divided doses and, in addition, the upper limit can also be exceeded when this is found to be indicated.

The following examples illustrate the present invention without limiting it, but serve merely as representative thereof. The pharmaceutical compositions conveniently contain about 1-500 mg, particularly 1-100 mg, of a compound of formula (I). The pharmaceutical compositions conveniently contain about 1-500 mg, particularly 1-100 mg, of a compound of formula (II). In certain embodiments the pharmaceutical compositions containing a compound of formula (I) contains in addition about 1-500 mg, particularly 1-100 mg, of a MEK inhibitor in a fixed-dose combination.

Non-limiting examples of compositions according to the invention are:

### Example A

Tablets of the following composition are manufactured in the usual manner:

**Table 3: possible tablet composition**

| ingredient | mg/tablet | | | |
|---|---|---|---|---|
| | 5 | 25 | 100 | 500 |
| Compound of formula (I) | 5 | 25 | 100 | 500 |
| Lactose Anhydrous DTG | 125 | 105 | 30 | 150 |
| Sta-Rx 1500 | 6 | 6 | 6 | 60 |
| Microcrystalline Cellulose | 30 | 30 | 30 | 450 |
| Magnesium Stearate | 1 | 1 | 1 | 1 |
| Total | 167 | 167 | 167 | 831 |

### Manufacturing Procedure

1. Mix ingredients 1, 2, 3 and 4 and granulate with purified water.
2. Dry the granules at 50°C.
3. Pass the granules through suitable milling equipment.
4. Add ingredient 5 and mix for three minutes; compress on a suitable press.

### Example B-1

Capsules of the following composition are manufactured:

**Table 4: possible capsule ingredient composition**

| ingredient | mg/capsule | | | |
|---|---|---|---|---|
| | 5 | 25 | 100 | 500 |
| Compound of formula (I) | 5 | 25 | 100 | 500 |
| Hydrous Lactose | 159 | 123 | 148 | - |
| Corn Starch | 25 | 35 | 40 | 70 |
| Talk | 10 | 15 | 10 | 25 |
| Magnesium Stearate | 1 | 2 | 2 | 5 |
| Total | 200 | 200 | 300 | 600 |

### Manufacturing Procedure

1. Mix ingredients 1, 2 and 3 in a suitable mixer for 30 minutes.
2. Add ingredients 4 and 5 and mix for 3 minutes.
3. Fill into a suitable capsule.

The compound of formula (I), lactose and corn starch are firstly mixed in a mixer and then in a comminuting machine. The mixture is returned to the mixer; the talc is added thereto and mixed thoroughly. The mixture is filled by machine into suitable capsules, e.g. hard gelatin capsules.

### Example B-2

Soft Gelatin Capsules of the following composition are manufactured:

**Table 5: possible soft gelatin capsule ingredient composition**

| ingredient | mg/capsule |
|---|---|
| Compound of formula (I) | 5 |
| Yellow wax | 8 |
| Hydrogenated Soya bean oil | 8 |
| Partially hydrogenated plant oils | 34 |
| Soya bean oil | 110 |
| Total | 165 |

**Table 6: possible soft gelatin capsule composition**

| ingredient | mg/capsule |
|---|---|
| Gelatin | 75 |
| Glycerol 85 % | 32 |
| Karion 83 | 8 (dry matter) |
| Titan dioxide | 0.4 |
| Iron oxide yellow | 1.1 |
| Total | 116.5 |

### Manufacturing Procedure

The compound of formula (I) is dissolved in a warm melting of the other ingredients and the mixture is filled into soft gelatin capsules of appropriate size. The filled soft gelatin capsules are treated according to the usual procedures.

### Example C

Suppositories of the following composition are manufactured:

**Table 7: possible suppository composition**

| ingredient | mg/supp. |
|---|---|
| Compound of formula (I) | 15 |
| Suppository mass | 1285 |
| Total | 1300 |

### Manufacturing Procedure

The suppository mass is melted in a glass or steel vessel, mixed thoroughly and cooled to 45°C. Thereupon, the finely powdered compound of formula (I) is added thereto and stirred until it has dispersed completely. The mixture is poured into suppository moulds of suitable size, left to cool; the suppositories are then removed from the moulds and packed individually in wax paper or metal foil.

### Example D

Injection solutions of the following composition are manufactured:

**Table 8: possible injection solution composition**

| ingredient | mg/injection solution. |
|---|---|
| Compound of formula (I) | 3 |
| Polyethylene Glycol 400 | 150 |
| acetic acid | q.s. ad pH 5.0 |
| water for injection solutions | ad 1.0 ml |

### Manufacturing Procedure

The compound of formula (I) is dissolved in a mixture of Polyethylene Glycol 400 and water for injection (part). The pH is adjusted to 5.0 by acetic acid. The volume is adjusted to 1.0 ml by addition of the residual amount of water. The solution is filtered, filled into vials using an appropriate overage and sterilized.

### Example E

Sachets of the following composition are manufactured:

**Table 9: possible sachet composition**

| ingredient | mg/sachet |
|---|---|
| Compound of formula (I) | 50 |
| Lactose, fine powder | 1015 |
| Microcrystalline cellulose (AVICEL PH 102) | 1400 |
| Sodium carboxymethyl cellulose | 14 |
| Polyvinylpyrrolidon K 30 | 10 |
| Magnesium stearate | 10 |
| Flavoring additives | 1 |
| Total | 2500 |

### Manufacturing Procedure

The compound of formula (I) is mixed with lactose, microcrystalline cellulose and sodium carboxymethyl cellulose and granulated with a mixture of polyvinylpyrrolidone in water. The granulate is mixed with magnesium stearate and the flavoring additives and filled into sachets.

### Abbreviations

CAS = chemical abstracts service; DCM = dichloromethane; DIPEA = N,N-diisopropylethylamine; DMF = dimethylformamide; DMSO = dimethyl sulfoxide; DNA = deoxyribonucleic acid; EDC·HCl = 1-(3-dimethylaminopropyl)-3-ethylcarbodiimide hydrochloride; ESI = electrospray ionization; EtOAc = ethyl acetate; HOOBt = 3,4-dihydro-3-hydroxy-4-oxo-1,2,3-benzotriazine; LC-MS/MS = liquid chromatography-MS/MS; MeOH = methanol; MS = mass spectrometry; NMP = N-methyl-2-pyrrolidone; PCR = polymerase chain reaction; rt = room temperature; SFC = supercritical fluid chromatography; THF = tetrahydrofuran.

### Test agents

(3*R*)-*N*-[2-cyano-4-fluoro-3-(3-methyl-4-oxo-quinazolin-6-yl)oxy-phenyl]-3-fluoro-pyrrolidine-1-sulfonamide (herein referred to as Compound Ia) was provided as a powder from Roche, Basel, Switzerland and resuspended prior to use. 2-(4-Cyclopropyl-2-fluoroanilino)-3,4-difluoro-5-[[3-fluoro-2-(methylsulfamoylamino)pyridin-4-yl]methyl]benzamide sodium salt (herein referred to as Compound IIa) was provided as a powder from Chugai, Tokyo, Japan. Cobimetinib (cat# HY-13064A), Encorafenib (cat # HY-15605) and Binimetinib (cat# HY-15202 ) were purchased from MedChemExpress

### Cell line and culture conditions

Cell lines were obtained from ATCC, maintained in humidified incubators at 5% CO2 in standard conditions and passaged twice a week. Culture conditions are reported In the following table:

| Cell line | catalog#/ origin | culture condition |
|---|---|---|
| A375 | CRL-1619 | Dulbecco's Modified Eagle's Medium, high glucose, GlutaMAX (DMEM GIBCO #10566016), 10% Fetal Bovine Serum (FBS, GIBCO #10270-106) |
| A375-NRAS | CRL-1619IG-2 | Dulbecco's Modified Eagle's Medium, high glucose, GlutaMAX (DMEM GIBCO #10566016), 10% Fetal Bovine Serum (FBS, GIBCO #10270-106) |

### Animals:

In the in vivo studies, female mice 7-9 weeks old (at experiment initiation) were purchased from Charles River Laboratories. The strain utilized in the experiments was CB.17 SCID.

For xenograft establishment cells were suspended in a media composed of 50% Matrigel and 50% Hank's balanced Salt Solution and injected subcutaneously on the right flanks. When tumor volume reached around 100 mm³ mice were randomized prior treatment.

### Examples

The following examples and figures are provided to illustrate the invention and have no limiting character.

### Example 1

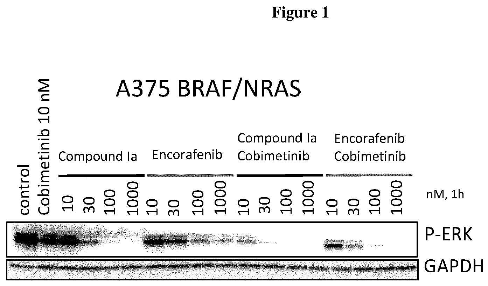

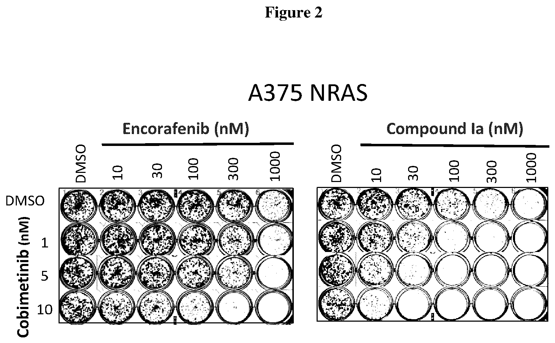

The A375 cell line presenting BRAF V600E and the RAF dimer inducing mutation NRAS Q61K was utilized to model a mechanism of resistance to BRAFi and BRAFi/MEKi combinations. A375 NRAS Q61K cells were treated with either Compound Ia, Encorafenib alone or in combination with 10nM of the MEKi Cobimetinib for 1 h and then utilized for Western Blot analysis of phosphorylated ERK, which is also termed as P-ERK (Figure 1). Similarly, A375 NRAS Q61K cells were plated at 500 cells /well, treated with either Compound Ia, Encorafenib alone or in combination with the MEKi Cobimetinib and incubated for 12 days. Colonies derived were fixed and stained with a solution of crystal violet/methanol (Figure 2). According to its paradox breaker property, Compound Ia drives superior P-ERK inhibition compared to Encorafenib and correspondingly combination of Compound Ia with Cobimetinib results in superior P-ERK repression compared to what triggered by the combination Encorafenib/Cobimetinib.

### Example 2

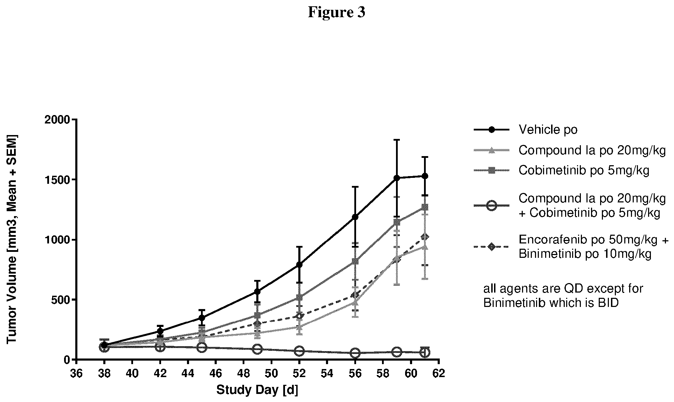

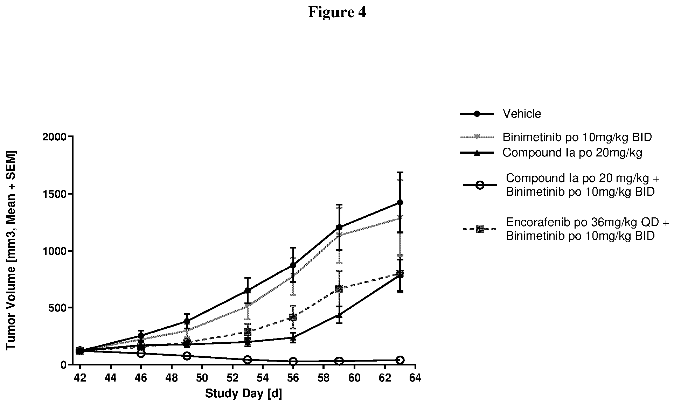

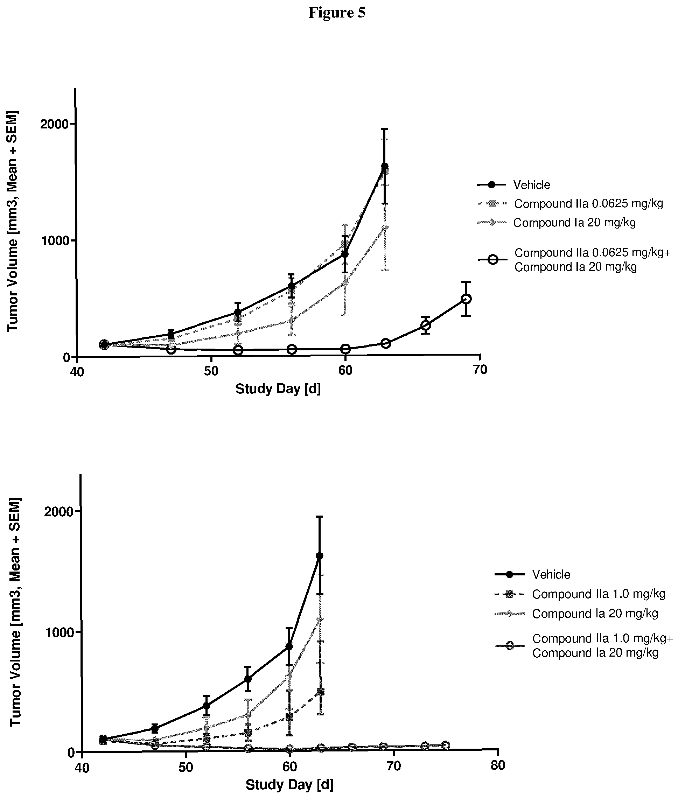

Immuodeficient mice were implanted with the cell line A375 NRAS presenting BRAF V600E and the RAF dimer inducing mutation NRAS Q61K as model of resistance to first generation BRAFi and BRAFi/MEKi. Upon tumor establishment (100mm3) mice were randomized and received orally (PO) once per day (QD) either Compound Ia (20 mg/kg), its combination with the MEKi cobimetinib (5 mg/kg, QD PO) or cobimetinib alone (Figure 3). The same mice model was also treated with Compound Ia (20 mg/kg) once per day in combination with a different MEK inhibitor binimetinib (10 mg/kg, BID PO) (Figure 4). For comparison, the animals of one of the experimental arms were treated with the paradox inducing BRAFi encorafenib (36 mg/kg, QD PO) in combination with binimetinib, which is an FDA approved combination for the treatment of metastatic melanoma. The same mice model was also treated with Compound Ia (20 mg/kg) once per day in combination with a different MEK inhibitor Compound IIa (one cohort at 0.0625 mg/kg, PO; a second cohort at 1.0 mg/kg, PO) (Figure 5).

### The following Example (Example 3) is not according to the invention and is present for illustration purposes only

### Example 3

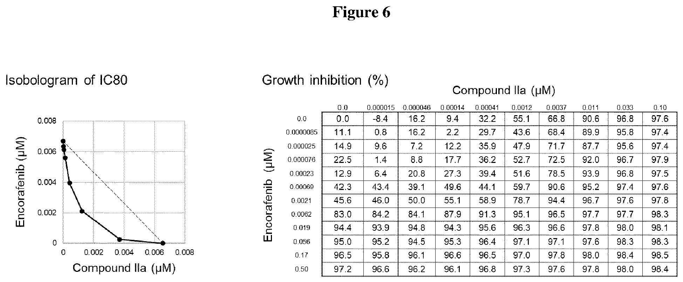

The A375 cell line was obtained from ATCC, maintained in humidified incubators at 5% CO2 in standard conditions. Cells were treated with Encorafenib and Compound IIa at indicated concentrations for 7 days on 384-well plates (U bottom). Cell viability was measured by CellTiter-Glo 2.0 (Promega, G9243) and the EnVision plate reader (Perkin Elmer). Cell growth inhibiton by compounds were calculated by the formula (1 - (T - V0)/(V - V0)) x 100 (%), where T represents the measured value of the wells with compounds, V represents the measured value of the wells without compounds and V0 represents the measured value of the wells without cells. Values for 80% inhibitory concentration (IC80) were calculated, and isobologram of IC80 was plotted. X- and Y-axes represent concentration of Compound IIa and Encorafenib, respectively. The isobole is hyperbolic and locaed below the additivity line (dashed line), indicating synergistic effects between Encorafenib and Compound IIa (Figure 6).

| Cell line | catalog#/ origin | culture condition |
|---|---|---|
| A375 | CRL-1619 | DMEM, high glucose (Sigma-Aldrich, D5796), 10% FBS (Corning, 35-076-CV), 1 mM sodium pyruvate (GIBCO, 11360-070) |

## Claims

1. A combination of a BRAF inhibitor and a MEK inhibitor wherein the BRAF inhibitor is a compound of formula (I) 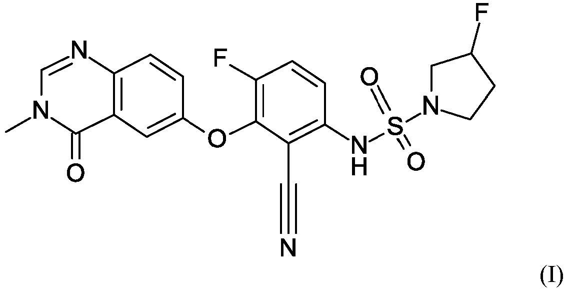
or a pharmaceutically acceptable salt or solvate thereof, and
wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or pharmaceutically acceptable salts thereof.

2. A combination of a BRAF inhibitor and a MEK inhibitor according to claim 1, wherein the compound of formula (I) is (3R)-N-[2-cyano-4-fluoro-3-(3-methyl-4-oxo-quinazolin-6-yl)oxy-phenyl]-3-fluoro-pyrrolidine-1-sulfonamide.

3. A combination of a BRAF inhibitor and a MEK inhibitor according to any one of claims 1 to 2, wherein the MEK inhibitor is 2-(4-cyclopropyl-2-fluoroanilino)-3,4-difluoro-5-[[3-fluoro-2-(methylsulfamoylamino)pyridin-4-yl]methyl]benzamide or a pharmaceutically acceptable salt thereof.

4. A combination of a BRAF inhibitor and a MEK inhibitor according to any one of claims 1 to 2, wherein the MEK inhibitor is cobimetinib or a pharmaceutically acceptable salt thereof.

5. A combination of a BRAF inhibitor and a MEK inhibitor according to any one of claims 1 to 4, for use as a medicament.

6. A combination of a BRAF inhibitor and a MEK inhibitor according to any one of claims 1 to 4, for use in the therapeutic and/or prophylactic treatment of cancer.

7. A pharmaceutical composition comprising a combination of a BRAF inhibitor and a MEK inhibitor according to any one of claims 1 to 4 and one or more pharmaceutically acceptable excipients.

8. A combination for use according to claim 6 or a pharmaceutical composition according to claim 7, wherein the BRAF inhibitor and the MEK inhibitor are both administered orally.

9. A combination for use according to claims 6 or 8, or a pharmaceutical composition according to claims 7 or 8, wherein the BRAF inhibitor is administered concurrently with the MEK inhibitor.

10. A combination for use according to claims 6, 8 or 9, or a pharmaceutical composition according to claims 7, 8 or 9, wherein the BRAF inhibitor and the MEK inhibitor are co-formulated.

11. A combination for use, according to claims 6 or 8, or a pharmaceutical composition according to claims 7 or 8, wherein the BRAF inhibitor is administered sequentially with the MEK inhibitor.

12. A combination of a BRAF inhibitor and a MEK inhibitor for use according to claim 6, wherein the cancer is thyroid cancer, colorectal cancer, melanoma, brain cancer or non-small cell lung cancer.

13. A combination of a BRAF inhibitor and a MEK inhibitor for use according to claim 6, wherein the cancer is associated with BRAF^{V600} mutations.

14. A combination of a BRAF inhibitor and a MEK inhibitor for use according to claim 6, wherein the cancer is BRAF^{V600} mutation-positive unresectable or metastatic cancer.

15. A combination of a BRAF inhibitor and a MEK inhibitor for use according to claims 13 or 14, wherein BRAF^{V600} mutation is determined using a method comprising (a) performing PCR or sequencing on nucleic acid (e.g., DNA) extracted from a sample of the patient's tumour tissue and/or body fluid; and (b) determining expression of BRAF^{V600} in the sample.

16. A combination of a BRAF inhibitor and a MEK inhibitor for use according to claim 6, comprising one or more additional anticancer agents selected from MEK degraders, EGFR inhibitors, EGFR degraders, inhibitors of HER2 and/or HER3, degraders of HER2 and/or HER3, SHP2 inhibitors, SHP2 degraders, Axl inhibitors, Axl degraders, ALK inhibitors, ALK degraders, PI3K inhibitors, PI3K degraders, SOS1 inhibitors, SOS1 degraders, signal transduction patway inhibitors, checkpoint inhibitors, modulators of the apoptosis pathway, cytotoxic chemotherapeutics, angiogenesis-targeted therapies, immune-targeted agents, and antibody-drug conjugates.

17. A compound for use in the treatment or prophylaxis of cancer in combination with a MEK inhibitor, wherein the compound is a compound of formula (I) 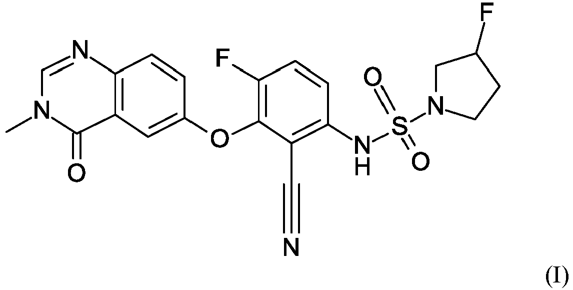
or a pharmaceutically acceptable salt or solvate thereof, and
wherein the MEK inhibitor is selected from 2-(4-cyclopropyl-2-fluoroanilino)-3,4-difluoro-5-[[3-fluoro-2-(methylsulfamoylamino)pyridin-4-yl]methyl]benzamide, cobimetinib, trametinib and binimetinib, or from a pharmaceutically acceptable salt or solvate thereof.

18. A compound for use according to claim 17, wherein the compound of formula (I) is a compound according to formula (Ia)

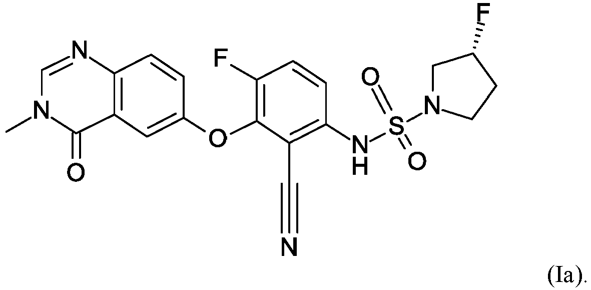

19. A compound for use according to claim 17 or 18, wherein the cancer is selected from thyroid cancer, colorectal cancer, melanoma, brain cancer and non-small cell lung cancer.

## Patentansprüche

1. Kombination eines BRAF-Inhibitors und eines MEK-Inhibitors, wobei der BRAF-Inhibitor eine Verbindung der Formel (I) 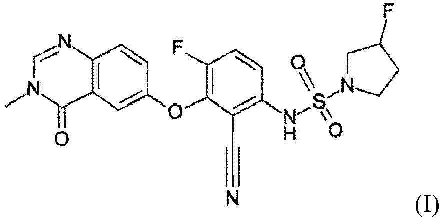
oder ein pharmazeutisch annehmbares Salz oder Solvat davon ist, und
wobei der MEK-Inhibitor aus 2-(4-Cyclopropyl-2-fluoranilino)-3,4-difluor-5-[[3-fluor-2-(methylsulfamoylamino)pyridin-4-yl]methyl]benzamid, Cobimetinib, Trametinib und Binimetinib oder pharmazeutisch annehmbaren Salzen davon ausgewählt ist.

2. Kombination eines BRAF-Inhibitors und eines MEK-Inhibitors nach Anspruch 1, wobei die Verbindung der Formel (I) (3R)-N-[2-Cyano-4-fluor-3-(3-methyl-4-oxochinazolin-6-yl)oxyphenyl]-3-fluorpyrrolidin-1-sulfonamid ist.

3. Kombination eines BRAF-Inhibitors und eines MEK-Inhibitors nach einem der Ansprüche 1 bis 2, wobei der MEK-Inhibitor 2-(4-Cyclopropyl-2-fluoranilino)-3,4-difluor-5-[[3-fluor-2-(methylsulfamoylamino)pyridin-4-yl]methyl]benzamid oder ein pharmazeutisch annehmbares Salz davon ist.

4. Kombination eines BRAF-Inhibitors und eines MEK-Inhibitors nach einem der Ansprüche 1 bis 2, wobei der MEK-Inhibitor Cobimetinib oder ein pharmazeutisch annehmbares Salz davon ist.

5. Kombination eines BRAF-Inhibitors und eines MEK-Inhibitors nach einem der Ansprüche 1 bis 4 zur Verwendung als Medikament.

6. Kombination eines BRAF-Inhibitors und eines MEK-Inhibitors nach einem der Ansprüche 1 bis 4 zur Verwendung bei der therapeutischen und/oder prophylaktischen Behandlung von Krebs.

7. Pharmazeutische Zusammensetzung, umfassend eine Kombination eines BRAF-Inhibitors und eines MEK-Inhibitors nach einem der Ansprüche 1 bis 4 und einen oder mehrere pharmazeutisch annehmbare Hilfsstoffe.

8. Kombination zur Verwendung nach Anspruch 6 oder pharmazeutische Zusammensetzung nach Anspruch 7, wobei der BRAF-Inhibitor und der MEK-Inhibitor beide oral verabreicht werden.

9. Kombination zur Verwendung nach Anspruch 6 oder 8 oder pharmazeutische Zusammensetzung nach Anspruch 7 oder 8, wobei der BRAF-Inhibitor gleichzeitig mit dem MEK-Inhibitor verabreicht wird.

10. Kombination zur Verwendung nach Anspruch 6, 8 oder 9 oder pharmazeutische Zusammensetzung nach Anspruch 7, 8 oder 9, wobei der BRAF-Inhibitor und der MEK-Inhibitor co-formuliert sind.

11. Kombination zur Verwendung nach Anspruch 6 oder 8 oder pharmazeutische Zusammensetzung nach Anspruch 7 oder 8, wobei der BRAF-Inhibitor nacheinander mit dem MEK-Inhibitor verabreicht wird.

12. Kombination eines BRAF-Inhibitors und eines MEK-Inhibitors zur Verwendung nach Anspruch 6, wobei der Krebs Schilddrüsenkrebs, Kolorektalkrebs, Melanom, Gehirnkrebs oder nichtkleinzelliger Lungenkrebs ist.

13. Kombination eines BRAF-Inhibitors und eines MEK-Inhibitors zur Verwendung nach Anspruch 6, wobei der Krebs mit BRAF^{V600}-Mutationen assoziiert ist.

14. Kombination eines BRAF-Inhibitors und eines MEK-Inhibitors zur Verwendung nach Anspruch 6, wobei der Krebs BRAF^{V600}-mutationspositiver, nicht resezierbarer oder metastatischer Krebs ist.

15. Kombination eines BRAF-Inhibitors und eines MEK-Inhibitors zur Verwendung nach Anspruch 13 oder 14, wobei BRAF^{V600}-Mutation unter Verwendung eines Verfahrens bestimmt wird, umfassend (a) Durchführen von PCR oder Sequenzierung an Nukleinsäure (z. B. DNA), die aus einer Probe des Tumorgewebes und/oder der Körperflüssigkeit des Patienten extrahiert wurde; und (b) Bestimmen der Expression von BRAF^{V600} in der Probe.

16. Kombination eines BRAF-Inhibitors und eines MEK-Inhibitors zur Verwendung nach Anspruch 6, umfassend ein oder mehrere zusätzliche Antikrebsmittel, ausgewählt aus MEK-Degradern, EGFR-Inhibitoren, EGFR-Degradern, Inhibitoren von HER2 und/oder HER3, Degradern von HER2 und/oder HER3, SHP2-Inhibitoren, SHP2-Degradern, Axl-Inhibitoren, Axl-Degradern, ALK-Inhibitoren, ALK-Degradern, PI3K-Inhibitoren, PI3K-Degradern, SOS1-Inhibitoren, SOS1-Degradern, Inhibitoren von Signaltransduktionswegen, Checkpoint-Inhibitoren, Modulatoren des Apoptosewegs, zytotoxischen Chemotherapeutika, auf Angiogenese gerichteten Therapien, auf Immunität gerichteten Mitteln und Antikörper-Arzneimittel-Konjugaten.

17. Verbindung zur Verwendung bei der Behandlung oder Prophylaxe von Krebs in Kombination mit einem MEK-Inhibitor, wobei die Verbindung eine Verbindung der Formel (I) 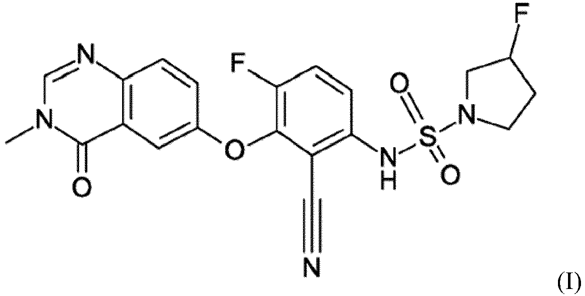
oder ein pharmazeutisch annehmbares Salz oder Solvat davon ist, und
wobei der MEK-Inhibitor aus 2-(4-Cyclopropyl-2-fluoranilino)-3,4-difluor-5-[[3-fluor-2-(methylsulfamoylamino)pyridin-4-yl]methyl]benzamid, Cobimetinib, Trametinib und Binimetinib oder aus einem pharmazeutisch annehmbaren Salz oder Solvat davon ausgewählt ist.

18. Verbindung zur Verwendung nach Anspruch 17, wobei die Verbindung der Formel (I) eine Verbindung der Formel (Ia) ist

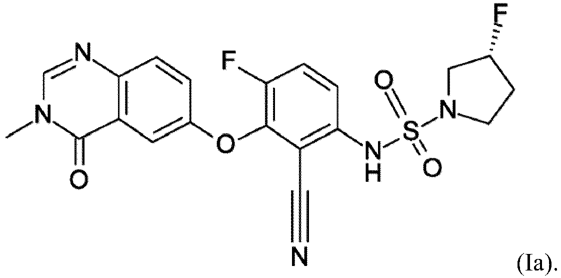

19. Verbindung zur Verwendung nach Anspruch 17 oder 18, wobei der Krebs aus Schilddrüsenkrebs, Kolorektalkrebs, Melanom, Gehirnkrebs und nichtkleinzelligem Lungenkrebs ausgewählt ist.

## Revendications

1. Combinaison d'un inhibiteur de BRAF et d'un inhibiteur de MEK dans laquelle l'inhibiteur de BRAF est un composé de formule (I) 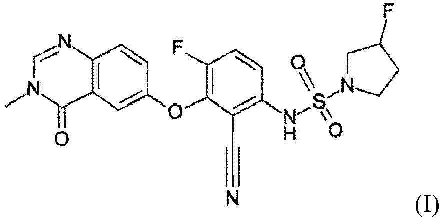
ou un sel pharmaceutiquement acceptable ou un solvate de celui-ci, et
dans laquelle l'inhibiteur de MEK est choisi parmi le 2-(4-cyclopropyl-2-fluoroanilino)-3,4-difluoro-5-[[3-fluoro-2-(méthylsulfamoylamino)pyridin-4-yl]méthyl]benzamide, le cobimétinib, le tramétinib et le binimétinib, ou des sels pharmaceutiquement acceptables de ceux-ci.

2. Combinaison d'un inhibiteur de BRAF et d'un inhibiteur de MEK selon la revendication 1, dans laquelle le composé de formule (I) est le (3R)-N-[2-cyano-4-fluoro-3-(3-méthyl-4-oxo-quinazolin-6-yl)oxy-phényl]-3-fluoro-pyrrolidine-1-sulfonamide.

3. Combinaison d'un inhibiteur de BRAF et d'un inhibiteur de MEK selon l'une quelconque des revendications 1 à 2, dans laquelle l'inhibiteur de MEK est le 2-(4-cyclopropyl-2-fluoroanilino)-3,4-difluoro-5-[[3-fluoro-2-(méthylsulfamoylamino)pyridin-4-yl]méthyl]benzamide ou un sel pharmaceutiquement acceptable de celui-ci.

4. Combinaison d'un inhibiteur de BRAF et d'un inhibiteur de MEK selon l'une quelconque des revendications 1 à 2, dans laquelle l'inhibiteur de MEK est le cobimétinib ou un sel pharmaceutiquement acceptable de celui-ci.

5. Combinaison d'un inhibiteur de BRAF et d'un inhibiteur de MEK selon l'une quelconque des revendications 1 à 4, pour une utilisation comme médicament.

6. Combinaison d'un inhibiteur de BRAF et d'un inhibiteur de MEK selon l'une quelconque des revendications 1 à 4, pour une utilisation dans le traitement thérapeutique et/ou prophylactique du cancer.

7. Composition pharmaceutique comprenant une combinaison d'un inhibiteur de BRAF et d'un inhibiteur de MEK selon l'une quelconque des revendications 1 à 4 et un ou plusieurs excipients pharmaceutiquement acceptables.

8. Combinaison pour une utilisation selon la revendication 6 ou composition pharmaceutique selon la revendication 7, dans laquelle l'inhibiteur de BRAF et l'inhibiteur de MEK sont tous deux administrés par voie orale.

9. Combinaison pour une utilisation selon les revendications 6 ou 8, ou composition pharmaceutique selon les revendications 7 ou 8, dans laquelle l'inhibiteur de BRAF est administré de manière simultanée à l'inhibiteur de MEK.

10. Combinaison pour une utilisation selon les revendications 6, 8 ou 9, ou composition pharmaceutique selon les revendications 7, 8 ou 9, dans laquelle l'inhibiteur de BRAF et l'inhibiteur de MEK sont coformulés.

11. Combinaison pour une utilisation selon les revendications 6 ou 8 ou composition pharmaceutique selon les revendications 7 ou 8, dans laquelle l'inhibiteur de BRAF est administré de manière séquentielle avec l'inhibiteur de MEK.

12. Combinaison d'un inhibiteur de BRAF et d'un inhibiteur de MEK pour une utilisation selon la revendication 6, dans laquelle le cancer est un cancer de la thyroïde, un cancer colorectal, un mélanome, un cancer du cerveau ou un cancer du poumon non à petites cellules.

13. Combinaison d'un inhibiteur de BRAF et d'un inhibiteur de MEK pour une utilisation selon la revendication 6, dans laquelle le cancer est associé à des mutations BRAF^{V600}.

14. Combinaison d'un inhibiteur de BRAF et d'un inhibiteur de MEK pour une utilisation selon la revendication 6, dans laquelle le cancer est un cancer non résécable ou métastatique positif aux mutations BRAF^{V600}.

15. Combinaison d'un inhibiteur de BRAF et d'un inhibiteur de MEK pour une utilisation selon les revendications 13 ou 14, dans laquelle la mutation BRAF^{V600} est déterminée en utilisant une méthode comprenant (a) la réalisation d'une PCR ou d'un séquençage sur l'acide nucléique (p. ex. l'ADN) extrait d'un échantillon d'un tissu tumoral et/ou d'un fluide corporel du patient ; et (b) la détermination de l'expression de BRAF^{V600} dans l'échantillon.

16. Combinaison d'un inhibiteur de BRAF et d'un inhibiteur de MEK pour une utilisation selon la revendication 6, comprenant un ou plusieurs agents anticancéreux supplémentaires choisis parmi les agents de dégradation de MEK, les inhibiteurs d'EGFR, les agents de dégradation d'EGFR, les inhibiteurs de HER2 et/ou HER3, les agents de dégradation de HER2 et/ou HER3, les inhibiteurs de SHP2, les agents de dégradation de SHP2, les inhibiteurs d'Axl, les agents de dégradation d'Axl, les inhibiteurs d'ALK, les agents de dégradation d'ALK, les inhibiteurs de PI3K, les agents de dégradation de PI3K, les inhibiteurs de SOS1, les agents de dégradation de SOS1, les inhibiteurs de voie de transduction du signal, les inhibiteurs de point de contrôle, les modulateurs de la voie de l'apoptose, les agents chimiothérapeutiques cytotoxiques, les thérapies ciblant l'angiogenèse, les agents ciblant le système immunitaire et les conjugués anticorps-médicament.

17. Composé pour une utilisation dans le traitement ou la prophylaxie d'un cancer en combinaison avec un inhibiteur de MEK, dans lequel le composé est un composé de formule (I) 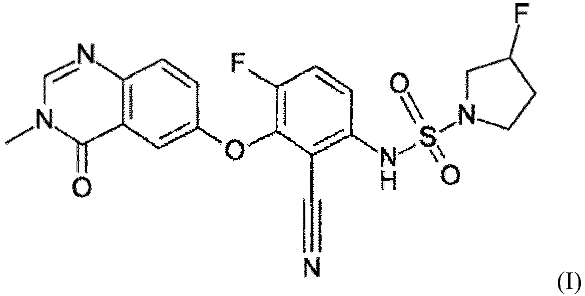
ou un sel pharmaceutiquement acceptable ou un solvate de celui-ci, et
dans lequel l'inhibiteur de MEK est choisi parmi le 2-(4-cyclopropyl-2-fluoroanilino)-3,4-difluoro-5-[[3-fluoro-2-(méthylsulfamoylamino)pyridin-4-yl]méthyl]benzamide, le cobimétinib, le tramétinib et le binimétinib, ou parmi un sel pharmaceutiquement acceptable ou un solvate de ceux-ci.

18. Composé pour une utilisation selon la revendication 17, dans lequel le composé de formule (I) est un composé selon la formule (Ia)

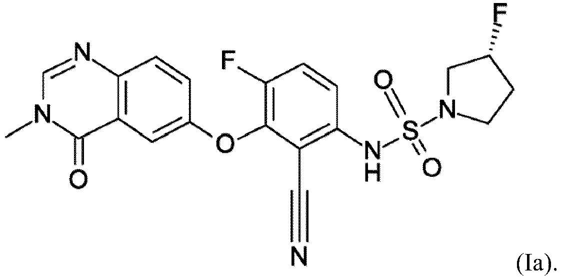

19. Composé pour une utilisation selon la revendication 17 ou 18, dans lequel le cancer est choisi parmi un cancer de la thyroïde, un cancer colorectal, un mélanome, un cancer du cerveau et un cancer du poumon non à petites cellules.
